# EUROPEAN PATENT SPECIFICATION

(11) **EP 2 755 977 B1**
(45) Date of publication and mention of the grant of the patent: **02.11.2016**
(21) Application number: 12775124.6
(22) Date of filing: 13.09.2012
(51) Int. Cl.: C07D 473/34, A61K 31/52, A61K 31/522, A61P 35/00, A61P 3/00, A61P 7/00, A61P 19/00, A61P 29/00, A61P 37/00, A61Q 19/00, A61K 8/49

(54) **6,8-DISUBSTITUTED PURINE COMPOSITIONS AND THEIR PHARMACEUTICAL AND COSMETIC USE**
6, 8-DISUBSTITUIERTE PURINZUSAMMENSETZUNGEN SOWIE IHRE PHARMAZEUTISCHE UND KOSMETISCHE VERWENDUNG
COMPOSITIONS DE PURINES 6,8-DISUBSTITUÉES ET LEUR UTILISATION PHARMACEUTIQUE ET COSMÉTIQUE

(30) Priority: 16.09.2011 US 201161535751 P
(43) Date of publication of application: 23.07.2014
(73) Proprietor: Univerzita Palackého v Olomouci, 771 47 Olomouc (CZ)
(72) Inventor: ZAHAJSKA, Lenka, 412 01 Litomerice (CZ); HANUS, Jan, 152 00 Praha 5 (CZ); SPICHAL, Lukas, 772 00 Olomouc (CZ); VOLLER, Jiri, 635 00 Brno-Bystrc (CZ); STRNAD, Miroslav, 779 00 Olomouc (CZ); ZATLOUKAL, Marek, 787 01 Sumperk (CZ)
(74) Representative: Hartvichova, Katerina
(86) International application number: PCT/CZ2012/000092
(87) International publication number: WO 2013/037333

(56) References cited:
- WO-A1-01/49688
- WO-A2-2007/125315
- US-A- 3 498 970
- US-A- 5 021 422
- US-A- 5 371 089
- US-A1- 2008 009 508
- US-A1- 2009 312 319
- MIKHAIL YU. STEKLOV ET AL: "Facile Synthesis of 8-Azido-6-Benzylaminopurine", NUCLEOSIDES, NUCLEOTIDES & NUCLEIC ACIDS, vol. 30, no. 7-8, 1 July 2011 (2011-07-01) , pages 503-511, XP055048693, ISSN: 1525-7770, DOI: 10.1080/15257770.2011.602655
- TAYLOR J L ET AL: "Biotransformation of adenine and cytokinins by the rhizobacterium Serratia proteamaculans", PHYTOCHEMISTRY, PERGAMON PRESS, GB, vol. 67, no. 17, 1 September 2006 (2006-09-01), pages 1887-1894, XP028059818, ISSN: 0031-9422, DOI: 10.1016/J.PHYTOCHEM.2006.06.016 [retrieved on 2006-09-01]
- R DELIGT ET AL: "Synthesis and biological evaluation of disubstituted N6-cyclopentyladenine analoguesthe search for a neutral antagonist with high affinity for the adenosine A1 receptor", BIOORGANIC & MEDICINAL CHEMISTRY, vol. 12, no. 1, 2 January 2004 (2004-01-02), pages 139-149, XP055048694, ISSN: 0968-0896, DOI: 10.1016/S0968-0896(03)00699-0
- HEONJOONG K ET AL: "Aplidiamine, a Unique Zwitterionic Benzyl Hydroxyadenine from the Western Australian Marine Ascidian Aplidiopsis sp", TETRAHEDRON LETTERS, ELSEVIER, AMSTERDAM, NL, vol. 38, no. 6, 10 February 1997 (1997-02-10), pages 941-944, XP004033901, ISSN: 0040-4039, DOI: 10.1016/S0040-4039(97)00003-8
- CHONG-MAW CHEN ET AL: "Biosynthesis and cytokinin activity of 8-hydroxy and 2,8-dihydroxy derivatives of zeatin and N 6 -([Delta] 2 -isopentenyl)adenine", BIOCHEMISTRY, vol. 14, no. 14, 1 July 1975 (1975-07-01), pages 3088-3093, XP055048703, ISSN: 0006-2960, DOI: 10.1021/bi00685a008
- LAURENCE G. DAMMANN ET AL: "Cytokinins: Synthesis of 2-, 8-, and 2,8-substituted 6-(3-methyl-2-butenylamino)purines and their relative activities in promoting cell growth", PHYTOCHEMISTRY, vol. 13, no. 2, 1 February 1974 (1974-02-01), pages 329-336, XP055048706, ISSN: 0031-9422, DOI: 10.1016/S0031-9422(00)91213-5
- R. MORNET ET AL: "Active Cytokinins: Photoaffinity Labeling Agents to Detect Binding", PLANT PHYSIOLOGY, vol. 64, no. 4, 1 October 1979 (1979-10-01), pages 600-610, XP055048755, ISSN: 0032-0889, DOI: 10.1104/pp.64.4.600
- DATABASE CA [Online] CHEMICAL ABSTRACTS SERVICE, COLUMBUS, OHIO, US; LETTRE, HANS ET AL: "Specific action of 6-purylhistamine on cancer cells", XP002689788, retrieved from STN Database accession no. 1968:48119 & LETTRE, HANS ET AL: "Specific action of 6-purylhistamine on cancer cells", NATURWISSENSCHAFTEN , 55(1), 43 CODEN: NATWAY; ISSN: 0028-1042, 1968,
- Roland K. Robins: "Potential Purine Antagonists. XV. Preparation of Some 6,8-Disubstituted Purines 1", Journal of the American Chemical Society, vol. 80, no. 24, 1 December 1958 (1958-12-01), pages 6671-6679, XP055195888, ISSN: 0002-7863, DOI: 10.1021/ja01557a051
- DATABASE REGISTRY [Online] CHEMICAL ABSTRACTS SERVICE, COLUMBUS, OHIO, US 19 April 2011 Database accession no. RN: 1282106-37-9

## Description

### Field of Art

The present invention relates to novel 6,8-disubstituted purines, their use and compositions comprising these compounds.

### Background Art

Natural cytokinins are adenine derivatives and can be classified by the configuration of their N⁶-side chain as isoprenoid or aromatic cytokinins. Cytokinins with an unsaturated isoprenoid side chain are by far the most prevalent, in particular those with a trans-hydroxylated N⁶-side chain, trans-zeatin (Letham, Life Sci, 8:569-573 (1963); Shaw et al., Proc. Chem. Soc. p.231 (1964)) and its derivatives. Dihydrozeatin, the counterpart of zeatin with a saturated side chain, has been identified in many species, while cis-zeatin and N⁶-(isopentenyl)adenine (i6Ade) are generally minor components although exceptions exist (Durand et al., 295-304 (1994;) Emery et al., Plant Physiol. 117: 1515-23 (1998)). Kinetin and N⁶-benzyladenine (BA) are the best-known cytokinins with ring substitutions at the N⁶-position. In the early years of cytokinin research, only cytokinins with an isoprenoid side chain were thought to be endogenous compounds, however, in the mid-1970s BA derivatives were identified as natural cytokinins (Horgan et al., Phytochemistry 14, 1005-8 (1975); Horgan et al., Tetrahederon Lett. 30:2827-2828 (1973)). The phenylureas constitute a group of synthetic cytokinins, some of which are highly active, (e.g., CPPU (N-phenyl-N-2-chloro-4-pyridyl urea) (Takahashi et al., Phytochemistry 17: 1201-1207 (1978)) and thidiazuron (Mok et al.: Phytochemistry 21:1509-1511 (1982)). Cytokinins were discovered in the search for factors that promoted division of plant cells in culture. Naturally occurring cytokinins are N⁶-substituted adenine derivatives that generally contain an isoprenoid derivative side chain. These hormones influence numerous aspects of plant development and physiology, including seed germination, de-etiolation, chloroplast differentiation, apical dominance, plant-pathogen interactions, flower and fruit development, and leaf senescence. These processes are also influenced by various other stimuli (e.g., light and other phytohormones) and the physiological and developmental outcomes reflect a highly integrated response to these multiple stimuli. Since all living organisms on the Earth have been evolutionary developing together for many millions of years, the presence of regulatory interactions of plant compounds, as cytokinins are, in animals and human can be assumed. Cytokinin-derived compounds probably affect many different molecular mechanisms in animal and human cells. 6-(substituted amino)purines (e.g., kinetin and zeatin) and 6,9-disubstituted purines have been shown to have biological activities related to aging. See, for example, U.S. Patents 5,021,422 (June 4, 1991), 5,371,089 (December 6, 1994), 5,602,139 (February 11, 1997), 5,614,407 (March 25,407), and U.S. Patent Publication 2008/0009508 (January 10, 2008) . It still remains desirable to provide additional compounds which can be used in a wide variety of therapeutic and cosmetic applications, especially for compounds having improved selectivities and efficiencies and lower toxicities than the known 6-(substituted amino) purines and 6,9-disubstituted purines.

### Disclosure of the Invention

This invention provides 6,8-disubstituted purines and compositions containing 6,8-disubstituted purines; these compounds and compositions possess significant biological activities.

The 6,8-disubstituted purines of this invention are represented by the general formula 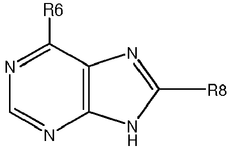 and include their salts;
wherein R6 is -NH-R_{y},
R_{y} is selected from the group consisting of furfuryl, benzyl, 3-methylbut-2-en-1-yl, wherein R_{y} can be unsubstitued or substituted with one or more hydroxy, methoxy, and
R8 is selected from the group consisting of amino, amino(C₁-C₅ alkyl)amino, methoxy, alkyloxycarbonyl, and alkylamino.

Throughout this specification, unless specifically indicated otherwise, the terms "general formula," "the general formula," or "6,8-disubstituted purine(s)" will refer to the chemical formula given in the above paragraph. Throughout this specification, unless specifically indicated otherwise, the generic substituents used in this specification for the 6,8-disubstituted purine(s) have the following meanings:
- hydroxy denotes the group -OH;
- amino denotes the group -NH₂;
- alkoxy denotes the group -OR_{c}, wherein R_{c} is alkyl as defined herein;
- alkylamino denotes the group -N(Rₐ)₂, wherein each Rₐ is independently selected from hydrogen, alkyl, and alkenyl as defined herein and which can be substituted by amino or hydroxyl substituent;
- alkyloxycarbonyl denotes the group -C(O)ORₑ, wherein Rₑ is alkyl or alkenyl as defined herein;
- alkyl denotes a branched or unbranched alkyl chain containing 1 to 8 carbon atoms,
- alkenyl denotes a branched or unbranched alkenyl chain containing 2 to 7 carbon atoms (preferably vinyl, allyl, 1-propenyl, 1-methylethenyl, but-1 to 3-enyl, pent-1 to 4-enyl, hex-1 to 5-enyl, hept-1 to 6-enyl, 3-methylbut-2-en-1-yl).

The salts of the 6,8-disubstituted purines useful in the present invention include, for example, alkali metal salts, ammonium salts, amine salts, and addition salts with acids; such salts may be in the form of racemates or optically active isomers. Preferably, the salts are pharmaceutically or cosmetically acceptable salts.

These 6,8-disubstituted purines have a wide range of biological activities, including for example anti-inflammatory, anti-senescent, pro-differentiation as well as well as other activities which are especially useful in pharmaceutical and cosmetic applications. The 6,8-disubstituted purine compounds and compositions containing such 6,8-disubstituted purines provide growth-regulatory, differentiating, antisenescent and antiaging properties with improved selectivities and efficiencies and lower toxicities than analogues known heretofore.

The preferred 6,8-disubstituted purines of this invention are 8-amino-6-(R_{y}-NH)purines wherein R_{y} is furfuryl, benzyl, which can be optionally substituted with hydroxy, methoxy, or combinations thereof.

In a preferred embodiment, R_{y} is selected from the group consisting of furfuryl, benzyl, 3-methylbut-2-en-1-yl, wherein the selected R_{y} can be unsubstituted or substituted with one or more hydroxy, methoxy, or combinations thereof.

In another preferred embodiment, R8 is selected from the group consisting of amino, amino(C₁-C₅ alkyl)amino, and methoxy.

The following derivatives are particularly preferred, namely: 6-furfurylamino-8-(amino, , amino(C₁-C₅ alkyl)amino, methoxy)purine, 6-(2-methoxybenzylamino)-8-(amino, amino(C₁-C₅ alkyl)amino, methoxy)purine, 6-(3-methoxybenzylamino)-8-(amino, amino(C₁-C₅ alkyl)amino, methoxy)purine, 6-(2-hydroxybenzylamino)-8-(amino, amino(C₁-C₅ alkyl)amino, methoxy)purine, 6-(3-hydroxybenzylamino)-8-(amino, amino(C₁-C₅ alkyl)amino, methoxy)purine, 6-(4-hydroxybenzylamino)-8-(amino, amino(C₁-C₅ alkyl)amino, methoxy)purine, 6-(3,4-dihydroxybenzylamino)-8-(amino, amino(C1-C5 alkyl)amino, methoxy)purine, 6-(3,5-dihydroxybenzylamino)-8-(amino, amino(C₁-C₅ alkyl)amino, methoxy)purine, 6-(3,5-dimethoxybenzylamino)-8-(amino, amino(C₁-C₅ alkyl)amino, methoxy)purine, 6-(2,5-dimethoxybenzylamino)-8-(amino, amino(C₁-C₅ alkyl)amino, methoxy)purine, 6-(3-hydroxy-4-methoxybenzylamino)-8-(amino, amino(C₁-C₅ alkyl)amino, methoxy)purine, 6-(2-hydroxy-3-methoxybenzylamino)-8-(amino, amino(C₁-C₅ alkyl)amino, methoxy)purine, 6-(4-hydroxy-3-methoxybenzylamino)-8-(amino, amino(C₁-C₅ alkyl)amino, methoxy)purine, 6-(2-hydroxy-5-methoxybenzylamino)-8-(amino, amino(C₁-C₅ alkyl)amino, methoxy)purine, 6-(3,4,5-trimethoxybenzylamino)-8-(amino, amino(C₁-C₅ alkyl)amino, methoxy)purine, 6-(Z)-(4-hydroxy-3-methylbut-2-en-1-ylamino)-8-(amino, amino(C₁-C₅ alkyl)amino, methoxy)purine, 6-(E)-(4-hydroxy-3-methylbut-2-en-1-ylamino)-8-(amino, amino(C₁-C₅ alkyl)amino, methoxy)purine.

Generally, the most preferred 6,8-disubstituted purine is 6-furfurylamino-8-aminopurine having the structure 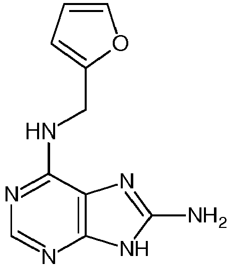 and which has a furfurylamino group at the 6-position and a -NH₂ group at the 8-position. This invention provides antisenescent and cell stimulatory compounds having improved selectivity and efficiency index (i.e., they are generally less toxic yet more efficacious than analogues known heretofore). The 6,8-disubstituted purines and compositions of this invention can, for example, assist in the regulation of cyclin-dependent kinases (CDKs), CDC25 phosphatases, cell proliferation, cell differentiation, cell apoptosis, and the like and can also interact with cytokinin receptors.

Described are methods for regulating CDKs, CDC25 phosphatases and cell proliferation and/or for inducing differentiation in an organism, comprising administering an effective amount of a composition comprising one or more compounds of this invention to the organism. The CDK/CDC25 regulating compounds are useful for treating disorders, some of them involving transcription, cell proliferation, differentiation or apoptosis, and thus are useful as antiproliferative treatment agents.

Described are pharmaceutical compositions comprising one or more 6,8-disubstituted purines in a pharmaceutically acceptable carrier system.

This invention further provides cosmetic compositions comprising one or more 6,8-disubstituted purines in a cosmetically acceptable carrier system.

This invention also provides methods for inhibiting cell senescence and aging in mammals or plants comprising application of an effective amount of the 6,8-disubstituted purines using either *in vivo* or *in vitro* techniques, according to claims 6 and 9.

This invention also provides methods for inhibiting or delaying the adverse effects of aging and/or improving the cosmetic appearance of mammalian cells, especially human skin cells, by applying an effective amount of the 6,8-disubstituted purines to the mammalian cells.

Described are methods for stimulation of cell proliferation and/or differentiation in an organism by application of an effective amount of the 6,8-disubstituted purines of this invention.

Described are pharmaceutical compositions comprising one or more 6,8-disubstituted purines of the present invention in a mixture with one or more pharmaceutical excipients.

This invention also provides cosmetic compositions comprising one or more 6,8-disubstituted purines of the present invention in mixtures with one or more cosmetically acceptable carriers.

Compositions containing one or more of the 6,8-disubstituted purines of the present invention are useful for treating senescing and aging cells in mammals and plants. Compositions containing one or more of the 6,8-disubstituted purines of the present invention are useful as anti-inflammatory and immunosuppressive treatment regimes.

The 6,8-disubstituted purines of the present invention can also be used as growth regulators in tissue cultures for stimulation of proliferation, morphogenesis, and senescence.

This invention also provides cosmetic compositions comprising one or more 6,8-disubstituted purines of this invention or their cosmetically and/or pharmaceutically acceptable salts thereof with alkali metals, ammonium or amines, in the forms of racemates or optically active isomers, or their addition salts with acids, with one or more carriers which can be used for reducing or ameliorating one or more adverse effects of aging. This invention includes a method for improving the appearance of human skin by topically applying an effective amount to the skin. As used herein, ameliorating the adverse effect of aging of mammalian cells means that the development of the morphological changes that normally occur with aging in normal mammalian cells *in vitro* or *in vivo* is slowed, reversed, and/or delayed. The adverse effects of aging also include age related changes in gene expression and protein biosynthesis. The ameliorative effect referred to herein is achieved without substantially increasing the growth rate or total proliferative capacity of the cells that are treated. Ameliorating the adverse effects of aging on cells may be detected as a delay or reversal of the onset of age-related morphological and phenotypical changes that normally occur with aging of the cells. Age related changes *in vivo* include changes in mammalian tissues, such as the development of, or increase in number or depth of, wrinkles, lines, sagging skin, discolorations, blotchiness, leathery, and/or yellowed appearance associated with the cosmetic appearance of the skin as well as the associated changes in the structural and functional integrity of the tissue. The 6,8-disubstituted purines of this invention are effective in improving the overall appearance and condition of the skin, including age-related changes and changes that may not be closely related to aging (e.g., acne, erythema, redness, and the like). For purposes of this invention, such changes that may not be closely related to aging or may even be independent of aging are intended to be included in age-related changes. Improvements in cosmetic appearance includes slowing, reversing, or stopping the development of undesirable cosmetic features, or otherwise improving the cosmetic appearance of the skin. The present invention can thus be used to improve the cometic appearance of human skin by reducing, or making less visible or less noticeable, the number or depth of wrinkles or lines, or reducing sagging skin, discolorations, blotchiness, leathery, and/or yellowed appearance of the skin such that the skin has a more youthful or appealing appearance.

The 6,8-disubstituted purines of the present invention appear to have extremely high potency levels for mammals, especially humans, for a wide range of medical and cosmetic related conditions, especially relating to aging of cells including human skin cells.

The heterocyclic substituted 6,8-disubstituted purines can also be used as cell division and differentiation factors of plant, mammal, microorganisms, yeast, and fungal cells.

The heterocyclic substituted 6,8-disubstituted purines of this invention have been found useful in cosmetics for treatment of humans for a wide variety conditions, especially of skin conditions which result in a negative or undesired appearance thereof. These compounds can also be used in pharmaceutical applications. Generally such uses will involve inclusion of the 6,8-disubstituted purines, or their appropriate salts in acceptable cosmetic or pharmaceutical carriers suitable for human use.

The compounds of this invention can also be used for preparation of affinity absorption matrices, immobilised enzymes for process control, immunoassay reagents, diagnostic samples, as well as compounds and oligonucleotides, labelled by ¹⁴C, ³H, avidin, biotin, or the like.

Pharmaceutical uses of these 6,8-disubstituted purines and their salts include, for example, use as mitotic or antimitotic compounds, especially for treating psoriasis, rheumatoid arthritis, lupus, type I diabetes, multiple sclerosis, restenosis, polycystic kidney disease, graft rejection, graft versus host disease and gout, parasitoses such as those caused by fungi or protists, or Alzheimer's disease, or as antineurodegenerative drugs, or to suppress immunostimulation.

Cosmetic uses of these 6,8-disubstituted purines and their salts include, for example, inhibition, delaying, or reducing the adverse effects of aging and senescence cells, especially human epidermal cells such as, for example, keratinocytes or fibroblasts. Thus, this invention especially provides methods for improving the cosmetic appearance of human skin; improvements in cosmetic appearance include, for example, reducing, or making less visible or less noticeable, the number or depth of wrinkles or lines, or reducing sagging skin, discolorations, blotchiness, leathery, and/or yellowed appearance of the skin such that the skin has a more youthful or appealing appearance.

The compounds of this invention, and especially the 6,8-disubstitued purine containing heterocyclic substitutents, can also be used for preparation of compositions suitable for use with plant and mammalian embryonic stem cells and embryo (especially oocytes) cloning.

The compounds of this invention, and especially the 6,8-disubstitued purine containing herterocyclic substitutents, can also be used for suppressing or controlling immunostimulation (e.g., arthritis or suppression of transplant rejection) in mammals.

Both the method of administration and dosage levels will, of course, depend on the condition being treated and its severity, the overall health of the patient, the site of the condition, as well as many other considerations. One of ordinary skill in the art, using the guidance provided herein, can easily determine the route of administration and dosage rates for a given patient whether for therapeutic or cosmetic application.

Suitable routes for administration include oral, rectal, topical (including dermal, ocular, buccal and sublingual), vaginal and parenteral (including subcutaneous, intramuscular, intravitreous, intravenous, intradermal, intrathecal and epidural).

The therapeutic and/or cosmetic compositions generally comprise about 1% to about 95% of the active ingredient. Single-dose forms of administration preferably comprise about 20% to about 90% of the active ingredient and administration forms which are not single-dose preferably comprise about 5% to about 20% of the active ingredient. Unit dose forms are, for example, coated tablets, tablets, ampoules, vials, suppositories or capsules. Other forms of administration are, for example, ointments, creams, pastes, foams, tinctures, lipsticks, drops, sprays, dispersions and the like. Examples are capsules containing from about 0.05 g to about 1.0 g of the active ingredient.

The pharmaceutical and cosmetic compositions of the present invention are prepared using standard techniques including, among others, conventional mixing, granulating, coating, dissolving, and/or lyophilising processes.

Preferably, solutions of the active ingredient, and in addition also suspensions or dispersions, especially isotonic aqueous solutions, dispersions or suspensions, are used, it being possible for these to be prepared before use, for example in the case of lyophilised compositions which comprise the active substance by itself or together with a carrier, for example mannitol. The compositions can be sterilized and/or comprise excipients, for example, preservatives, stabilisers, wetting agents and/or emulsifiers, solubilizing agents, salts for regulating the osmotic pressure and/or buffers, and they are prepared in a manner known *per se*, for example by means of conventional dissolving or lyophilising processes. The solutions or suspensions mentioned can comprise viscosity-increasing substances, such as sodium carboxymethylcellulose, carboxymethylcellulose, dextran, polyvinylpyrrolidone or gelatine.

Suspensions in oil comprise, as the oily component, vegetable, synthetic or semi-synthetic oils customary for injection purposes. Oils which may be mentioned are, in particular, liquid fatty acid esters which contain, as the acid component, a long-chain fatty acid having 8-22, in particular 12-22, carbon atoms (e., lauric acid, tridecylic acid, myristic acid, pentadecylic acid, palmitic acid, margaric acid, stearic acid, acid, arachidonic acid, behenic acid, and the like) or corresponding unsaturated acids (e.g., oleic acid, elaidic acid, euric acid, brasidic acid or linoleic acid). Other additional ingredients known in the art can be included if desired (e.g., antioxidants such as vitamin E, β-carotene, or 3,5-di-tert-butyl-4-hydroxytoluene, and the like). The alcohol component of these fatty acid esters generally contains no more than about 6 carbon atoms and can be mono- or polyhydric. Mono-, di-, or trihydric alcohols such as methanol, ethanol, propanol, butanol, or pentanol, or isomers thereof, can be used; glycols and glycerols are generally preferred. Fatty acid esters can therefore include, for example, ethyl oleate, isopropyl myristate, isopropyl palmitate, "Labrafil M 2375" (polyoxyethylene glycerol trioleate from Gattefoseé, Paris), "Labrafil M 1944 CS" (unsaturated polyglycolated glycerides prepared by an alcoholysis of apricot kernel oil and made up of glycerides and polyethylene glycol esters; from Gattefoseé, Paris), "Labrasol" (saturated polyglycolated glycerides prepared by an alcoholysis of TCM and made up of glycerides and polyethylene glycol esters; from Gattefoseé, Paris), and/or "Miglyol 812" (triglyceride of saturated fatty acids of chain length C8 to C12 from Hüls AG, Germany), and in particular vegetable oils, such as cottonseed oil, almond oil, olive oil, castor oil, sesame oil, soybean oil and, in particular, groundnut oil as well as mixtures thereof.

The preparation of the compositions intended for human use should, of course, be carried out in the customary and approved manner under sterile conditions, and maintained under appropriate conditions up to and including the time of use.

For example, pharmaceutical compositions for oral use can be obtained by combining the active ingredient with one or more solid carriers, if appropriate granulating the resulting mixture, and, if desired, processing the mixture or granules to tablets or coated tablet cores, if appropriate by addition of additional excipients. Suitable carriers are, in particular, fillers, such as sugars, for example lactose, sucrose, mannitol or sorbitol, cellulose preparations and/or calcium phosphates, for example tricalcium diphosphate, or calcium hydrogen phosphate, and furthermore binders, such as starches, for example maize, wheat, rice or potato starch, methylcellulose, hydroxypropylmethylcellulose, sodium carboxymethylcellulose and/or polyvinylpyrrolidone, and/or, if desired, desintegrators, such as the above mentioned starches, and furthermore carboxymethyl-starch, cross-linked polyvinylpyrrolidone, alginic acid or a salt thereof, such as sodium alginate. Additional excipients are, in particular, flow regulators and lubricants, for example salicylic acid, talc, stearic acid or salts thereof, such as magnesium stearate or calcium stearate, and/or polyethylene glycol, or derivatives thereof.

Coated tablet cores can be provided with suitable coatings which, if appropriate, are resistant to gastric juice, the coatings used being, *inter alia*, concentrated sugar solutions, which, if appropriate, comprise gum arabic, talc, polyvinylpyrrolidone, polyethylene glycol and/or titanium dioxide, coating solutions in suitable organic solvents or solvent mixtures or, for the preparation of coatings which are resistant to gastric juice, solutions of suitable cellulose preparations, such as acetylcellulose phthalate or hydroxypropylmethylcellulose phthalate. Dyes or pigments can be admixed to the tablets or coated tablet coatings, for example for identification or characterisation of different doses of active ingredient.

Pharmaceutical compositions, which can be used orally, can also be in the form hard capsules of gelatine and soft, closed capsules of gelatine and a plasticiser, such as glycerol or sorbitol. The hard capsules can contain the active ingredient in the form of granules, mixed for example with fillers, such as maize starch, binders and/or lubricants, such as talc or magnesium stearate, and stabilisers if appropriate. In soft capsules, the active ingredient is preferably dissolved or suspended in suitable liquid excipients, such as greasy oils, paraffin oil or liquid polyethylene glycol's or fatty acid esters of ethylene glycol or propylene glycol, it being likewise possible to add stabilisers and detergents such as, for example, the polyethylene sorbitan fatty acid ester type.

Other oral forms of administration include, for example, syrups prepared in the customary manner, which comprise the active ingredient, for example, in suspended form and in a concentration of about 5% to 20%, preferably about 10% or in a similar concentration which results in a suitable individual dose, for example, when 5 or 10 ml are measured out. Other forms include pulverulent or liquid concentrates for preparing shakes, beverages, and the like. Such concentrates can also be packed in unit dose quantities. Pharmaceutical compositions, which can be used rectally, are, for example, suppositories that comprise a combination of the active ingredient with a suppository base. Suitable suppository bases are, for example, naturally occurring or synthetic triglycerides, paraffin hydrocarbons, polyethylene glycols or higher alkanols.

Compositions which are suitable for parental administration are aqueous solutions of an active ingredient in water-soluble form, for example of water-soluble salt, or aqueous injection suspensions, which comprise viscosity-increasing substances, for example sodium carboxymethylcellulose, sorbitol and/or dextran, and if appropriate, stabilizers. The active ingredient can also be present here in the form of a lyophilisate, if appropriate together with excipients, and be dissolved before parenteral administration by addition of suitable solvents. Solutions such as are used, for example, for parental administration can also be used as infusion solutions. Preferred preservatives are, for example antioxidants, such as ascorbic acid, or microbicides, such as sorbic or benzoic acid.

Ointments are oil-in-water emulsions, which comprise not more than 70%, but preferably 20 - 50% of water or aqueous phase. The fatty phase consists, in particular, hydrocarbons, for example vaseline, paraffin oil or hard paraffin's, which preferably comprise suitable hydroxy compounds, such as fatty alcohol's or esters thereof, for example cetyl alcohol or wool wax alcohols, such as wool wax, to improve the water-binding capacity. Emulsifiers are corresponding lipophilic substances, such as sorbitan fatty acid esters (Spans), for example sorbitan oleate and/or sorbitan isostearate. Additives to the aqueous phase are, for example, humectants, such as polyalcohols, for example, glycerol, propylene glycol, sorbitol and/or polyethylene glycol, or preservatives and odoriferous substances.

Fatty ointments are anhydrous and comprise, as the base, in particular, hydrocarbons, for example paraffin, vaseline or paraffin oil, and furthermore naturally occurring or semi-synthetic fats, for example, hydrogenated coconut-fatty acid triglycerides, or, preferably, hydrogenated oils, for example hydrogenated groundnut or castor oil, and furthermore fatty acid partial esters of glycerol, for example glycerol mono- and/or distearate, and for example, the fatty alcohols. They also can contain emulsifiers and/or additives mentioned in connection with the ointments which increase uptake of water.

Creams are oil-in-water emulsions, which comprise more than 50% of water. Oily bases used are, in particular, fatty alcohols, for example, lauryl, cetyl or stearyl alcohols, fatty acids, for example palmitic or stearic acid, liquid to solid waxes, for example isopropyl myristate, wool wax or beeswax, and/or hydrocarbons, for example vaseline (petrolatum) or paraffin oil. Emulsifiers are surface-active substances with predominantly hydrophilic properties, such as corresponding non-ionic emulsifiers, for example fatty acid esters of polyalcohols or ethyleneoxy adducts thereof, such as polyglyceric acid fatty acid esters or polyethylene sorbitan fatty esters (Tweens), and furthermore polyoxyethylene fatty alcohol ethers or polyoxyethylene fatty acid esters, or corresponding ionic emulsifiers, such as alkali metal salts of fatty alcohol sulphates, for example, sodium lauryl sulphate, sodium cetyl sulphate or sodium stearyl sulphate, which are usually used in the presence of fatty alcohols, for example cetyl stearyl alcohol or stearyl alcohol. Additives to the aqueous phase are, *inter alia*, agents which prevent the creams from drying out, for example polyalcohols, such as glycerol, sorbitol, propylene glycol and/or polyethylene glycols, and furthermore preservatives and odoriferous substances.

Pastes are creams and ointments having secretion-absorbing powder constituents, such as metal oxides, for example, titanium oxide or zinc oxide, and furthermore talc and/or aluminium silicates, which have the task of binding the moisture or secretions present. Foams (i.e., liquid oil-in-water emulsions packaged in aerosol form) can be administered from pressurised containers. Propellant gases include halogenated hydrocarbons, such as polyhalogenated alkanes such as dichlorofluoromethane and dichlorotetrafluoroethane, or, preferably, non-halogenated gaseous hydrocarbons, air, N₂O, or carbon dioxide. The oily phases used are, *inter alia*, those mentioned above for ointments and creams, and the additives mentioned there are likewise used.

Tinctures and solutions usually comprise an aqueous-ethanolic base to which humectants for reducing evaporation, such as polyalcohols (e.g., glycerol, glycols, polyethylene glycol), and re-oiling substances, such as fatty acid esters with lower polyethylene glycols (e.g., lipophilic substances soluble in the aqueous mixture), to substitute the fatty substances removed from the skin with the ethanol, and, if necessary or desired, other excipients and additives are admixed.

The present invention further provides veterinary compositions comprising at least one active ingredient as above defined together with a veterinary carrier therefor. Veterinary carriers are materials for administering the composition and may be solid, liquid, or gaseous materials, which are inert or acceptable in the veterinary art and are compatible with the active ingredient. These veterinary compositions may be administered orally, parenterally, or by any other desired route.

It is also described a process or method for treatment of the disease states mentioned above. The compounds can be administered prophylactically or therapeutically as such or in the form of pharmaceutical compositions, preferably in an amount, which is effective against the diseases mentioned. With a warm-blooded animal, for example, a human requiring such treatment, the compounds are used, in particular, in the form of pharmaceutical composition. A daily dose of about 0.1 to about 5 g, preferably 0.5 g to about 2 g, of a compound of the present invention is administered here for a body weight of about 70 kg.

### Brief Description of Figures

Figure 1 shows charts of effect of several 6,8-disubstituted purines on retention of chlorophyll in excised wheat leaf tips. Values are expressed as % of the initial chlorophyll content of fresh leaves before incubation. Error bars show standard deviation of the mean for 5 replicate determinations. Control is the value for the treatment without any cytokinin.
Figure 2 shows charts of effect of several 6,8-disubstituted purines on fresh weight yield of tobacco callus culture. Error bars show standard deviation of the mean for 5 replicate determination. Control is the value for the treatment without any cytokinin.
Figure 3 shows effect of 6-furfurylamino-8-aminopurine on cell attachment.
Figure 4 shows effect of 6-furfurylamino-8-aminopurine on short-term growth.
Figure 5 shows effect of 6-furfurylamino-8-aminopurine on cell survival.
Figure 6 shows effect of 6-furfurylamino-8-aminopurine at several concentrations on lysosomal activity.
Figure 7 shows localization of JC-1 in ASF-2 cells using 6-furfurylamino-8-aminopurine at several concentrations by fluorescence microscopy. Figure 7a is a negative control showing lack of JC-1 aggregates. Figure 7b shows a control and 6-furfurylamino-8-aminopurine at various concentrations.
Figure 8 shows effect of 6-furfurylamino-8-aminopurine at several concentrations on proteasomal activity using ASP2 p11 (chymotrypsin like LLVY).
Figure 9 shows actin staining patterns after three days of treatment with 6-furfurylamino-8-aminopurine at several concentrations.
Figure 10 shows effect of 6-furfurylamino-8-aminopurine at several concentrations on the morphology of senescent cells after 7 and 14 days.
Figure 11 shows number of cells after 7 and 14 days of treatment with 6-furfurylamino-8-aminopurine at several concentrations.
Figure 12 is a line chart showing skin erythema scores in mouse skin assay for 6-furfurylamino-8-aminopurine and controls.
Figure 13 is a bar chart showing weekly erythema scores in mouse skin assay for 6-furfurylamino-8-aminopurine and controls.
Figure 14 shows skin moisture determinations in mouse assay for 6-furfurylamino-8-aminopurine and controls.
Figure 15 shows the content of moisture after 3 weeks in mouse assay for 6-furfurylamino-8-aminopurine and controls.
Figure 16 shows skin elasticity determinations in mouse assay for 6-furfurylamino-8-aminopurine and controls.
Figure 17 shows skin elasticity after 3 weeks in mouse assay for 6-furfurylamino-8-aminopurine and controls.
Figures 18a and 18b show microphotographs of bromodeoxyuridine staining for the inventive compound 6-furfurylamino-8-aminopurine and the tretinoin control, respectively.
Figure 19 shows histological evaluation of skin biopsies after 3 weeks of topical treatment with 6-furfurylamino-8-aminopurine and controls in mouse assay.
Figures 19a - 19g are identified as follows: 19a - control (untreated); 19b - control (vehicle); 19c - control (kinetin); 19d - control (zeatin & kinetin); 19e - control (zeatin); 19f - control (tretinoin); and 19g - control (6-furfurylamino-9-(2-tetrahydropyranyl)purine. Figure 19h is the inventive compound 6-furfurylamino-8-aminopurine.

### Examples of carrying out the Invention

The following examples contain also compounds that do not form part of the invention.

The following examples are included for illustrative purposes only and are not intended to limit the scope of the invention. Unless otherwise indicated, all concentrations, ratios, and the like are based on weight. The starting materials may be obtained from commercial sources (Sigma, Aldrich, Fluka, etc.) or can be prepared as described below.

Melting points were determined on a Koffler block and are uncorrected. Evaporations were carried out on a rotary evaporator under vacuum at temperatures below 80°C. The ¹H-NMR spectra (s, ppm; J, Hz) were measured on Varian VXR-400 (400MHz) or on Varian Unity 300 (400MHz) instruments. All spectra were obtained at 25°C using tetramethylsilane as internal standard. Electron impact mass spectra m/z (rel.%, composition, deviation) were measured on a VG 7070E spectrometer (70eV, 200°C, direct inlet). Quadrupole mass spectra were measured on a Micromass ZMD detector with electrospray ionization. Merck silica gel Kieselgel 60 (230-400 mesh) was used for column chromatography. All compounds gave satisfactory elemental analyses ( ±0.4%).

6,8-Disubstituted purines were prepared using the following procedures, including Methods A and B described below as well as other methods described in specific examples.
**Method A** - via 6,8-dihalogen-9-(2',3',5'-tri-*O*-acetyl-β-D-ribofuranosyl)purines. 6,8-dihalogen-9-substituted purines react with an nucleophile to afford a mixture of 6,9-disubstituted-8-halogen purines and 8,9-disubstituted-6-halogen purines which are separated by column chromatography. The desired isomer gives after hydrolysis the final compound or is remitted to next substitution. The locants R6 and R8 refer to the R6 and R8 specified above.
**Method B** - direct halogenation of R6-substiuted purines or R6,9-disubstituted purines. Bromine, N-bromosuccinimide or N-chlorosuccinimide could be used as halogenating agents. The halogen atom in the 8 position of 8-halogen-R6,9-substituted purines can be subjected to subsequent substitution by an appropriate nucleohile. R8 substituent could be further modified, if possible, for example by oxidation, hydrolysis or hydrogenation.

**Example 1.** This examples provides the preparation of several intermediates useful in method A:
1. 8-bromo-6-chloro-9-(2',3',5'-tri-*O*-acetyl-β-D-ribofuranosyl)purine. The reaction was carried out under argon atmosphere at laboratory temperature. Chlorotrimethylsilane (10.0 ml, 78.2 mmol) was added dropwise to the solution of 8-bromo-2',3',5'-tri-O-acetyladenosine (4.72 g, 10 mmol) in dichloromethane (75 ml). After short time, *terc-*butylnitrite (10.0 ml, 84.1 mmol) was slowly (1 drop/min) added dropwise. The mixture was then stirred for one hour at laboratory temperature placed to the refrigerator over night. The mixture was slowly under vigorous stirring poured into saturated NaHCO₃ solution. Layers were separated, the water layer was extracted with chloroform (20 ml). The combined organic layers were dried with Na₂SO₄, filtered and partly evaporated. The residue was purified by column chromatography (50 g of silica gel, mobile phase initially chloroform, then hexanes : ethyl acetate 1 : 1. Yield 2.79 g, 57 %. Light-yellowish crystals.
2. 6,8-Dichloro-9-(2',3',5'-tri-O-acetyl-β-D-ribofuranosyl)purine was prepared according to literature (Roelen et al., J. Med. Chem. 39, 1463, (1996)). Yield 94%. Structure of 6,8-dichloro-9-(2',3',5'-tri-O-acetyl-β-D-ribofuranosyl)purine was confirmed by identification of compounds prepared therefrom.
3. 8-Amino-6-chloro-9-(β-D-ribofuranosyl)purine was prepared according to literature (Szekeres et al., J. Heterocyclic. Chem. 12, 15, (1975)). Yield 50%. Mp: 214-217°C. MS ESI+:302.3 [M⁺H⁺]. NMR spectra were consistent with the literature.

**Example 2.** This example illustrates the preparation of several R6,R8-disubstituted-9-(β-D-ribofuranosyl)purines using Method A.

8-Amino-6-benzylamino-9-(ß-D-ribofuranosyl)purine. 6-Benzylamino-8-bromo-9-(B-D-ribofuranosyl)purine (43.3 mg, 0.099mmol) was dissolved in 6 ml MeOH saturated by NH₃ (saturated at 0°C) and solution was heated to boiling temperature for 48 h. The mixture was evaporated and residue was purified by column chromatography in CHCI₃-MeOH-NH₄OH (95:5:0.5). Yield 10.1 mg 8-amino-6-benzylamino-9-(ß-D-ribofuranosyl)purine (27%); mp = 80-82°C; MS ESI+: 373.3 [M+H⁺]; for C₁₇H₂₀N₆O₄ calculated 372.1546, found 373.1638 [M+H⁺]. ¹H NMR (300 MHz; *d₄*-CH₃OH) δ 3.75 (m, H5'), 3.94 (dd, *J*=2.4 Hz, 12.7 Hz, H5'), 4.22 (d, *J*=1.8 Hz, H4'), 4.29 (d, *J*=5.3 Hz, H3'), 4.63 (bs, -CH₂-), 5.08 (m, H2'), 6.12 (d, *J*=7.2 Hz, H1'), 7.33 (m, H-Ph), 8.27 (s, H2). Impurity 6-benzylamino-8-methoxy-9-(ß-D-ribofuranosyl)purine (51%); mp = 91-93°C. MS ESI+: 388.3 [M+H⁺]. For C₁₈H₂₁N₅O₅ calculated 387.1543, found 388.1599 [M+H⁺]. ¹H NMR (400 MHz;CDCl₃) δ 3.68 (m, H5'), 3.87 (dd, *J*=1.7 Hz, 12.9 Hz, H5'), 4.07 (s, OCH₃), 4.22 (m, H4'), 4.37 (d, *J*=5.0 Hz, H3'), 4.68 (dd, *J*=5.5 Hz, 14.7 Hz,-CH₂-), 4.84 (dd, *J*=6.2 Hz, 14.7 Hz, -CH₂-), 5.09 (dd, *J*=5.0 Hz, 7.5 Hz, H2'), 5.81 (dd, *J*=5.5 Hz, 6.2 Hz, H-N), 5.88 (d, *J*=7.5 Hz, H1'), 7.28 (m, H4-Ph), 7.33 (m, H3-Ph), 7.36 (m, H2-Ph), 7.97 (s, H2).

8-Amino-6-benzylamino-9-(ß-D-ribofuranosyl)purine. 8-Amino-6-chloro-9-(ß-D-ribofuranosyl)purine (92.9 mg, 0.308 mmol) was suspended in 2 ml n-propanol,135 µl benzylamine was added and the suspension was heated at 120°C for 14 h. The mixture was evaporated and residue was purified by column chromatography in CHCI₃-MeOH-NH₄OH (95:5:0.5). Yield 63 mg 8-amino-6-benzylamino-9-(ß-D-ribofuranosyl)purine (55%); mp = 78-82°C. MS ESI: 373.3 [M+H⁺].

8-Amino-6-furfurylamino-9-(ß-D-ribofuranosyl)purine. 8-Amino-6-chloro-9-(ß-D-ribofuranosyl)purine (146.1 mg, 0.484 mmol) was suspended in 2 ml n-propanol. Furfurylamine (0.5 ml) was added and suspension was heated to 120°C for 5 hours. The mixture was evaporated and the residue was purified by column chromatography in CHCI₃-MeOH-NH₄OH (9:1:0.1). Yield 28.8 mg of glassy 8-amino-6-furfurylamino-9-(ß-D-ribofuranosyl)purine (16%). MS ESI+: 363.3 [M+H⁺]. For C₁₅H₁₈N₆O₅ calculated 362.1339, found 363.1443 [M+H⁺]. ¹H-NMR (400 MHz, *d₆*-DMSO): 3.60 (d, *J*=12.1 Hz, H-5'a), 3.65 (dd, *J*=2.6 Hz, 12.1 Hz, H-5'b), 3.96 (m, H-4'), 4.13 (m, H-3'), 4.69 (m, H-2'), 4.71 (d, *J*=6.3 Hz, CH₂Fur), 5.90 (d, *J*=7.2 Hz, H-1'), 6.19 (dd, *J*=0.9 Hz, 3.2 Hz, Fur H-2), 6.34 (dd, *J*=1.8 Hz, 3.2 Hz, Fur H-3), 6.69 (bs, C⁸-NH₂, 7.23 (t, *J*=6.3 Hz, C⁶-NH), 7.52 (dd, *J*=0.9 Hz, 1.8 Hz, Fur H-4), 7.98 (s, C²-H).

8-Amino-6-(3-methylbut-2-en-1-ylamino)-9-(ß-D-ribofuranosyl)purine. 8-Amino-6-chloro-9-(ß-D-ribofuranosyl)purine (142.2 mg, 0,471 mmol) and 245.3 of hydrogen chlorid of 3-methylbut-2-en-1-ylamine was suspended in 3 ml n-propanol and suspension was heated to 120°C for 8 hours. The mixture was evaporated and the residue was purified by column chromatography in CHCI₃-MeOH-NH₄OH (95:5:0.5). Yield 39.2 mg of 8-amino-6-(3-methylbut-2-en-1-ylamino)-9-(ß-D-ribofuranosyl)purine (23%); mp: 121-124°C. MS ESI+: 351.3 [M+H⁺]. For C₁₅H₂₂N₆O₄ calculated 350.1703, found 351.1535 [M+H⁺]. ¹H NMR (400 MHz; *d₄*-CH₃OH) δ 1.75 (s, -CH₃), 3.76 (d, *J*=10.8 Hz, H5'), 3.84 (dd, *J*=12.3 Hz, H5'), 4.08 (d, *J*=6.0 Hz, H4'), 4.32 (dd, *J*=1.7 Hz, 5.5 Hz, H3'), 4.88 (bs, -CH₂-), 4.95 (d, *J*=9.1 Hz, H2'), 5.36 (bs, -CH=), 5.96 (d, *J*=7.3 Hz, H1'), 8.00 (s, H2).

6-benzylamino-8-chloropurine. 6,8-Dichloropurine (130.5 mg, 0.69 mmol) was dissolved in 1 ml of n-propanol. 222 mg (2.07 mmol) of benzylamine was added and the mixture was heated to 90°C for 3 hours. The solution was evaporated and the residue was purified by column chromatography in HCl₃-MeOH (97:3). Yield 92.6 mg of 6-benzylamino-8-chlorpurine (52%); mp: 237-238°C. MS ESI: 260 [M-H⁺]. For C₁₂H₁₆ClN₅ calculated: C 55.50%; H 3.88%; Cl 13.65%; N 26.97%; found: C 55.45%; H 4.09%; N 26.08%. ¹H-NMR (300 MHz; *d₆*-DMSO) δ 4.70 (s, -CH₂-), 7.25 (m, H-Ph), 8.18 (s, H2), 8.47 (bs, H9). 6-Benzylamino-8-bromopurine. 6-Benzylaminopurine (100 mg, 0.444 mmol) and N-bromosuccinimide (111 mg, 0.624 mmol) was dissolved in 1 ml. The solution was heated for 4.5 h at 50°C. The reaction mixture was then evaporated to dryness and then purified by column chromatography in CHCI₃-MeOH-NH₄OH (95:5:0.5). Yield of 28.4 mg 6-benzylamino-8-bromopurine (21%) and 48.1 mg starting material (48%); mp = 233-235°C. MS ESI+: 304.2 [M+H⁺]. For C₁₂H₁₀BrN₅ calculated 303.0120, found 304.0153 [M+H⁺]. ¹H-NMR (300 MHz; *d₆*-DMSO) δ 4.68 (s, -CH₂-), 7.25 (m, H-Ph), 8.14 (s, H2), 8.43 (bs, H9).

**Example 3.** This example illustrates the preparation of several R6,R8-disubstituted-9-(β-D-ribofuranosyl)purines using Method B.

6-Benzylamino-8-chloro-9-(ß-D-ribofuranosyl)purine. 6-Benzylamino-9-(ß-D-ribofuranosyl)purine (115 mg, 0.322 mmol) and 51.6 mg (0.386 mmol) N-chlorosuccinimide was dissolved in 3 ml DMF and the solution was heated at 45°C for 18 h. The mixture was evaporated and residue was purified by column chromatography in CHCI₃-MeOH-NH₄OH (95:5:0.5). Yield 25.2 mg 6-benzylamino-8-chloro-9-(ß-D-ribofuranosyl)purine (20%) a 62.1 mg starting compound (54%); mp = 90-92°C. MS ESI+: 392.2 [M+H⁺]. For C₁₇H₁₈ClN₈O₄ calculated 391.1047, found 392.1119 [M+H⁺]. ¹H NMR (300 MHz; CDCl₃) δ 3.61 (dd, *J*=4.1 Hz, 8.2 Hz, H5'), 3.89 (d, *J*=12.7 Hz, H5'), 4.27 (s, H4'), 4.45 (d, *J*=5.7 Hz, H3'), 4.74 (bs, -CH₂-), 4.79 (bs, -CH₂-), 5.00 (dd, *J*=5.7 Hz, 6.3 Hz, H2'), 6.01 (d, *J*=7.3 Hz, H1'), 6.38 (bs, H-N), 7.28 (m, H-Ph), 8.21 (s, H2). 8-Chloroadenosine was isolated as an analytical sample for MS ESI by wash out from TLC. MS ESI+: 302.3 [M+H⁺].

6-Benzylamino-8-bromo-9-(ß-D-ribofuranosyl)purine. 6-Benzylamino-9-(B-D-ribofuranosyl)purine (469.4 mg; 1.313 mmol) was suspended in 15 ml 1 M AcONa and 15 ml 1 M AcOH. Bromine water (12.7 ml) was added to suspension and mixture was heated for 2.5 h at 45°C. Excess bromine was eliminated by addition of solid NaHSO₃ and then the mixture was neutralized by 10% NaOH and evaporated. Residue was shaken out with water and chloroform. Organic layer was separated, dried in MgSO₄ and after filtration of desiccant evaporated to dryness. The residue was purified by column chromatography in CHCI₃-MeOH-NH₄OH (95:5:0.5). Yield 126.5 mg 6-benzylamino-8-bromo-9-(ß-D-ribofuranosyl)purine (22%), 42.2 mg starting material (9%), 205 mg 8-bromoadenosine (45%) and mixture of benzaldehyde and bromobenzaldehyde. Crystallization from CHCI₃-hexan; mp: 98-100°C. MS ESI+: 436.2 [M+H⁺]. For C₁₇H₁₃BrN₅O₄ calculated 435.0542, found 436.0680 [M+H⁺]. ¹H NMR (400 MHz; CDCl₃) δ 3.75 (dd, *J*=2.7 Hz, 12.7 Hz, H5'), 3.90 (dd, *J*=2.4 Hz, 12.7 Hz, H5'), 4.20 (d, *J*=1.8 Hz, H4'), 4.39 (dd, *J*=1.8 Hz, 5.3 Hz, H3'), 4.81 (bs, -CH₂-), 5.08 (dd, *J*=5.3 Hz, 7.2 Hz, H2'), 6.07 (d, *J*=7.2 Hz, H1'), 7.24 (m, H4-Ph), 7.31 (m, H3-Ph), 7.38 (m, H2-Ph), 8.19 (s, H2). MS ESI+ (8-bromoadenosine): 346.3 [M+H⁺].GC: Rₜ (benzaldehyde) = 321 s. MS EI (benzyldehyde): 105 (100%), 77 (25%). Rₜ (bromobenzyldehyde) = 722 s. MS EI: 185 (100%), 155 (50%), 77 (45%).

6-Benzylamino-8-dimethylamino-9-(ß-D-ribofuranosyl)purine. 6-Benzylamino-8-bromo-9-(ß-D-ribofuranosyl)purine (78 mg, 0.179 mmol) was dissolved in 3 ml 50% water-methanolic solution of dimethylamine (c = 22 mol/l) and then left to stand at laboratory temperature for 18 h. The mixture was evaporated and residue was purified by column chromatography in CHCI₃ : MeOH : NH₄OH (95:5:0.5). Yield 69.5 mg 6-benzylamino-8-dimethylamino-9-(ß-D-ribofuranosyl)purine (97%); crystallized from MeOH-Et₂O as hydrogenchloride; mp = 152-156°C. MS ESI: 401.3 [M+H⁺]. For Cl₉H₂₄N₆O₄ calculated 400.1859, found 401.1863 [M+H⁺]. ¹H NMR (300 MHz; *d₄*-CH₃OH) δ 3.11 (s, N(CH₃)₂), 3.73 (dd, *J*=2.7 Hz, 12.5 Hz, H5'), 3.86 (d, *J*=2.1 Hz, 12.5 Hz, H5'), 4.23 (d, *J*=2.0 Hz, H4'), 4.35 (d, *J*=6.0 Hz, H3'), 4.76 (m, -CH₂-), 5.10 (m, H2'), 6.00 (d, *J*=7.2 Hz, H1'), 7.24 (m, H-Ph), 8.21 (s, H2).

6-Benzylamino-8-methoxy-9-(ß-D-ribofuranosyl)purine. 6-Benzylamino-8-bromo-9-(ß-D-ribofuranosyl)purine (76.2 mg, 0.175 mmol) was dissolved in 2 ml dry MeOH supplemented by 4 ml abs. MeOH solution in which 104 mg Na were dissolved. The mixture was kept at laboratory temperature for 19 h, neutralized by AcOH, dried and residue was purified by column chromatography in CHCI₃-MeOH-NH₄OH (95:5:0.5). Yield 45.1 mg 6-benzylamino-8-methoxy-9-(ß-D-ribofuranosyl)purine (67%). MS ESI+: 388.2 [M+H⁺]. ¹H NMR (400 MHz; CDCl₃) δ 3.68 (1H, m, H5'), 3.87 (1H, dd, *J*=2.4 Hz, 12.7 Hz, H5'), 4.07 (3H, s, -OCH₃), 4.22 (1H, m, H4'), 4.37 (1H, d, *J*=5.3 Hz, H3'), 4.68 (1H, dd, *J*=5.5 Hz, 14.7 Hz, -CH₂-), 4.84 (1H, dd, *J*=6.2 Hz, 14.7 Hz, -CH₂-), 5.09 (dd, *J*=5.0 Hz, 7.5 Hz, H2'), 5.81 (1H, dd, *J*=5.5 Hz, 6.2 Hz, HN⁶), 5.88 (d, *J*=7.5 Hz, H1'), 7.28 (m, H4-Ph), 7.33 (m, H3-Ph), 7.36 (m, H2-Ph), 7.79 (s, H2).

6-Benzylamino-8-methoxy-9-(ß-D-ribofuranosyl)purine. 6-Benzylamino-8-methylsulfonyl-9-(ß-D-ribofuranosyl)purine (11.8 mg, 0.027mmol) was dissolved in 1 ml methanol and 0.1 ml NaOH and the solution was left at laboratory temperature for 1 h. The reaction mixture was evaporated and residue was purified by column chromatography in CHCI₃-MeOH-NH₄OH (95:5:0.5). Yield 10.1mg 6-benzylamino-8-methoxy-9-(ß-D-ribofuranosyl)purine (97%).MS ESI+: 388.2 [M+H⁺]. The 6-Benzylamino-8-methylsulfonyl-9-(ß-D-ribofuranosyl)purine (15.7 mg, 0.036 mmol) was dissolved in 2 ml methanol saturated by NH₃ (saturated at 0°C) and the solution was left at laboratory temperature for 18 h. The reaction mixture was evaporated and residue was purified by column chromatography in CHCl₃-MeOH-NH₄OH (95:5:0.5). Yield 6-benzylamino-8-methoxy-9-(ß-D-ribofuranosyl)purine 13.4 mg (96%). MS ESI+: 388.2 [M+H⁺].

8-(3-Aminopropylamino)-6-benzylamino-9-(ß-D-ribofuranosyl)purine. 6-Benzylamino-8-bromo-9-(ß-D-ribofuranosyl)purine (120 mg, 0.276 mmol) was dissolved in 2 ml of 2-propanol and 1 ml 1,3-diaminopropane (12 mmol) then left to stand at laboratory temperature for 24 h. The mixture was evaporated and residue was purified by column chromatography in CHCl₃:MeOH:NH₄OH (95:5:0.5). Yield 103 mg 8-(3-aminopropylamino)-6-benzylamino-9-(ß-D-ribofuranosyl)purine (87%); crystallized from MeOH-Et₂O; mp = 72-76°C. MS ESI: 430.5 [M+H⁺]. For C₂₀H₂₇N₇O₄ calculated 429.2125, found 430.3002 [M+H⁺]. ¹H NMR (400 MHz; *d₄*-CH₃OH) δ 1.79 (pent., *J*=5.6 Hz, -NH-CH₂-C**H₂**-CH₂-NH₂), 3.03 (m, -NH-(CH₂)₂-CH₂-NH₂), 3.67 (t, *J*=5.4 Hz, -NH-C**H₂**-(CH₂)₂-NH₂), 3.73 (dd, *J*=2.7 Hz, 12.5 Hz, H5'), 3.82 (bs, -NH-), 3.86 (d, *J*=2.1 Hz, 12.5 Hz, H5'), 4.23 (d, *J*=2.0 Hz, H4'), 4.35 (d, *J*=6.0 Hz, H3'), 4.76 (m, -CH₂-), 5.10 (m, H2'), 6.00 (d, *J*=7.2 Hz, H1'), 7.24 (m, H-Ph), 8.21 (s, H2).

**Example 4.** This example illustrates the preparation of several R6,R8-disubstituted-9-(β-D-ribofuranosyl)purines prepared by modification of R8 substituent.

6-Benzylamino-8-methoxycarbonyl-9-(ß-D-ribofuranosyl)purine. 6-Benzylamino-8-cyano-9-(ß-D-ribofuranosyl)purine (58.3 mg, 0.153 mmol) and 5.2 mg (0.046 mmol) of potassium *tert*-butoxide was dissolved in 3 ml abs. methanol. The solution was mixed for 18 hours at room temperature then cooled to -10°C, 1 ml of 1M HCI was added and mixed for 90 min. The solution was neutralized by sodium acetate and evaporated. The residue was extracted with water and CHCI₃. Organic layer was separated, dried with MgSO₄ and purified by column chromatography in CHCI₃-MeOH (95:5). Yield 39.8 mg of 6-benzylamino-8-methoxycarbonyl-9-(ß-D-ribofuranosyl)purine (62%) in the form of a foam. MS ESI+: 414.4 [M+H⁺]. For C₁₉H₂₁N₅O₆ calculated 475.1492, found 416.1576 [M+H⁺]. ¹H NMR (300 Hz; CDCl₃) 3.88 (m, H5'), 3.96 (s, **H**₃COOC), 4.28 (s, H4'), 4.50 (s, H3'), 4.84 (bs, -CH₂-), 5.00 (m, H2'), 5.32 (bs, H-N), 6.93 (d, *J*=6.7 Hz, H1'), 7.32 (m, H-Ph), 8.41 (s, H2). IR: 1731 cm⁻¹ (C=O).

**Example 5.** This example illustrates the preparation of several R6,R8-disubstituted-9-(β-D-ribofuranosyl)purines prepared via Dimroth rearrangement (Method C). R8,R9-disubstituted adenines are alkylated with an appropriate alkylhalogenide on the N¹ position of adenine ring. The intermediates are subsequently converted into R6,R8,R9-substituted purines under basic conditions.

6-benzylamino-8-bromo-9-(ß-D-ribofuranosyl)purine. 8-Bromoadenosine (210.7 mg, 0.609 mmol) was dissolved in 3 ml dry dimethylformamide and the mixture was then supplemented with 363 µl benzylbromide and 0.5 g molecular sieve 3Å. Reaction mixture was heated for 24 h at 50°C. The mixture was filtered and then 5 ml 26% NH₄OH was added; the reaction mixture was heated for 5 h at 50°C. The solution was extracted by CHCI₃, organic phase separated, dried by MgSO₄ and purified by column chromatography in CHCI₃-MeOH-NH₄OH (95:5:0.5). Yield 55.5 mg 6-benzylamino-8-bromo-9-(ß-D-ribofuranosyl)purine (21%) and 80 mg starting material (35%). Crystallization from CHCl₃-hexan; mp = 96-98°C. MS ESI+: 436.2 [M+H⁺]. ¹H NMR (400 MHz; CDCl₃) δ 3.75 (dd, *J*=2.7 Hz, 12.7 Hz, H5'), 3.90 (dd, *J*=2.4 Hz, 12.7 Hz, H5'), 4.20 (d, *J*=1.8 Hz, H4'), 4.39 (dd, *J*=1.8 Hz, 5.3 Hz, H3'), 4.81 (bs, -CH₂-), 5.08 (dd, *J*=5.3 Hz, 7.2 Hz, H2'), 6.07 (d, *J*=7.2 Hz, H1'), 7.24 (m, H4-Ph), 7.31 (m, H3-Ph), 7.38 (m, H2-Ph), 8.19 (s, H2).

8-Bromo-6-(3-methylbut-2-en-1-ylamino)-9-(ß-D-ribofuranosyl)purine. 8-Bromoadenosine (75.6 mg, 0.218 mmol) was dissolved in 3 ml dry DMF and 128 µl 1-bromo-3-methylbut-2-ene and 0.5 g molecular sieve 3A was added to the solution. Reaction mixture was heated at 50°C for 24 h. The mixture was filtered and then 5 ml 26% NH₄OH was added to the filtrate and mixture heated at 50°C for 5 h. The solution was extracted in CHCI₃, organic layer separated, dried by MgSO₄ and residue was purified by column chromatography in CHCI₃-MeOH-NH₄OH (95:5:0.5). Yield 17.8 mg glassy 8-bromo-6-(3-methylbut-2-en-1-ylamino)-9-(ß-D-ribofuranosyl)purine (18%). MS ESI+: 414.1 [M+H⁺]. For C₁₅H₂₀BrN₅O₄ calculated 413.0699, found 414.0805 [M+H⁺]. ¹H-NMR: ¹H NMR (400 MHz; *d₄*-CH₃OH) δ 1.76 (s, -CH₃), 3.73 (dd, *J*=2.9 Hz, 12.6 Hz, H5'), 3.89 (dd, *J*=2.3 Hz, 12.6 Hz, H5'), 4.17 (d, *J*=1.6 Hz, H4'), 4.36 (dd, *J*=1.8 Hz, 5.3 Hz, H3'), 4.88 (bs, -CH₂-), 5.04 (dd, *J*=5.3 Hz, 7.0 Hz, H2'), 5.36 (t, *J*=5.6 Hz, -CH=), 6.04 (d, *J*=7.0 Hz, H1'), 8.12 (s, H2).

**Example 6.** This example illustrates the preparation of several R6,R8-disubstituted-9-(β-D-ribofuranosyl)purines prepared via R8-2',3',5'-tri-O-acetylinosine (Method D). The O⁶ of R8 -2',3',5'-tri-O-acetylinosine can be replaced with alkylamino/aralkyamino group by alkylamine/aralkyamine under (benzotriazol-1-yloxy)tris(dimethylamino) phosphonium hexafluorophosphate catalysis.

Intermediates used in method D are prepared using the following procedures. 8-Bromo-2',3',5'-tri-O-acetylinosine was prepared by bromination of 2',3',5'-tri-O-acetylinosine with bromine in an aqueous Na₂HPO₄ according to literature (Roelen et al., J. Med. Chem. 39, 1463, (1996)). Yield 20%; purity: 98% (HPLC); mp: 192-195°C. 8-Methylsulfanylinosine. 8-Bromo-2',3',5'-tri-*O*-acetylinosine (0.89 g; 1.9 mmol) and sodium methanethiolate (0.60 g; 8.6 mmol) were dissolved in 10 ml dimethylformamide and stirred at room temperature over night. The solvent was evaporated. The residue was suspended in 12.0 ml acetonitrile. 1.2 ml (8.6 mmol) Triethylamine, 46.7 mg (0.38 mmol) dimethylaminopyridine and 810 µl (8.6 mmol) acetic anhydride were added to the suspension, and the mixture was stirred for 4 h at room temperature. The reaction was quenched by addition of CH₃OH (0.3 ml). The mixture was filtered, and the filtrate was concentrated using vacuum. The residue was purified by column chromatography (silica gel; mobile phase chloroform/acetone 7/3). Yellowish foam. Yield: 0.80 g, 97 %.

The preparation of R6,R8-disubstituted-9-(β-D-ribofuranosyl)purines prepared by method D is illustrated as follows. 6-(3-Methylbut-2-en-1-ylamino)-8-methylsulfanyl-9-(β-D-ribofuranosyl)purine was prepared from 8-methylsulfanylinosine applying the procedure described in literature (Wan, Binnun, Wilson, Lee, Org. Lett. **26,** 5877 (2005)). 8-Methylsulfanylinosine (42 mg; 96 µmol), 51 mg (116 µmol) (benzotriazol-1-yloxy)tris (dimethylamino)phosphonium hexafluorophosphate, 46µl (269 µmol) diisopropylethylamine were dissolved in 0.6 ml dimethylformamide. After stirring the solution for 10 min, 3-methyl-2-butenylamine hydrochloride was added. The reaction mixture was stirred at room temperature for 11 h and evaporated using vacuum. The residue was purified by column chromatography (silica gel; mobile phase chloroform/ethyl acetate 9/1). Yellowish foam. Yield: 24 mg, 51%. ¹H NMR is described above.

**Example 7.** In some cases, R6,R8-disubstituted-9-(β-D-ribofuranosyl)purines were used as intermediates (Method E) to prepare new inventive compounds by acid hydrolysis of 9-(β-D-ribofuranosyl) group using the following procedure: R6,R8-9-(β-D-ribofuranosyl)purine (0.1 mmol) was dissolved in 2 ml of 50% CF₃COOH (water solution). Solution was heated to 45°C for 3 h and then evaporated without neutralisation. The residue was purified by column chromatography in CHCI₃-MeOH-NH₄OH (95:5:0.5). The following compounds were prepared using this method.
(1) 8-Bromo-6-(3-methylbut-2-en-1-ylamino)purine was prepared from 8-bromo-6-(3-methylbut-2-en-1-ylamino)-9-(ß-D-ribofuranosyl)purine. Yield 18.2 mg of 8-bromo-6-(3-methylbut-2-en-1-ylamino)purine (65 %); mp: 180-182°C. MS ESI+: 283.1 [M+H⁺]. For C₁₀H₁₂BrN₅ calculated: C 42.57%; H 4.29%; Br 28.32%; N 24.83%; found: C 42.37%; H 4.11%; Br 28.33%, N 24.34%. ¹H NMR (300 MHz; *d₄*-CH₃OH) δ 1.75 (s, -CH₃), 4.88 (bs, -CH₂-), 5.36 (t, *J*=5.6 Hz, -CH=), 8.10 (s, H2), 8.40 (bs, H9).
(2) 6-Benzylamino-8-chloropurine was prepared from 6-benzylamino-8-chloro-9-(ß-D-ribofuranosyl)purine. Yield 18.2 mg of 6-benzylamino-8-chloropurine (67%); mp: 236-237°C. MS ESI-: 372.3 [M-H⁺]. For C₁₂H₁₆ClN₅ calculated: C 55.50%; H 3.88%; Cl 13.65%; N 26.97%; found: C 55.37%; H 4.11%; N 26.34%. ¹H NMR (300 MHz; *d₆*-DMSO) δ 4.70 (s, -CH₂-), 7.25 (m, H-Ph), 8.18 (s, H2), 8.47 (bs, H9).
(3) 6-Benzylamino-8-dimethylaminopurine was prepared from 6-benzylamino-8-dimethylamino-9-(ß-D-ribofuranosyl)purine. Yield 17 mg of 6-benzylamino-8-dimethylaminopurine (63%). Mp: 217-219°C. MS ESI+: 269.3 [M+H⁺]. For C₁₄H₁₆N₆ calculated 268.1436, found 269.1500 [M+H⁺]. ¹H NMR (300 MHz; *d₄*-CH₃OH) δ 3.11 (s, N(CH₃)₂), 4.71 (s, -CH₂-), 7.32 (m, H-Ph), 8.14 (s, H2).
(4) 8-Amino-6-benzylaminopurine was prepared from 8-amino-6-benzylamino-9-(ß-D-ribofuranosyl)purine (49.4 mg, 0.133 mmol). Yield 16.1 mg of 8-amino-6-benzylaminopurine (50%); mp: 223-226°C. MS ESI+: 241.3 [M+H⁺]. For C₁₂H₁₂N₆ calculated 240.1123, found 241.1136 [M+H⁺]. ¹H NMR (300 MHz; *d₄*-CH₃OH) δ 4.71 (s, -CH₂-), 7.30 (m, H-Ph), 8.06 (s, H2).
(5) 8-Amino-6-furfurylaminopurine was prepared by dissolving 8-Amino-6-furfurylamino-9-(ß-D-ribofuranosyl)purine (3.38g; 9.3mmol) in 50% trifluoroacetic acid at 0°C. The mixture was then warmed up to 50°C and stirred at this temperature for 12 h. Water (30ml) was then added into the dark solution. The final solution was stirred with activated charcoal (0.2g) for 5 minutes; the activated charcoal was removed by filtration. The filtration cake was washed with water (20ml). Combined filtrates were evaporated to give dark semi crystalline residue, which was dissolved in water (30ml). This solution was alkalized by 25% aqueous ammonia to pH = 8 (ca 12ml). The resulting thick crystalline suspension was then stirred for 1 h at temperature 5-10°C, and then filtered off. The off white crystalline product was washed with cold (5°C) water (2x10ml), and dried in dessiccator (phosphorus pentoxide/NaOH) into constant weight. Yield: 1.75g (81.5%). TLC (CHCl₃:MeOH:conc.NH₄OH (8:2:0.2, v/v/v)): single spot; HPLC purity: 99%+; mp: 128-130°C.
(6) 6-Benzylamino-8-methoxypurine was prepared from 6-benzylamino-8-methoxy-9-(ß-D-ribofuranosyl)purine applying the typical procedure. Yield 14 mg of 6-benzylamino-8-dimethylaminopurine (37%). Mp: 251-254°C. MS ESI+: 256,3 [M+H⁺]. For C₁₃H₁₃N₅O calculated 255.1120, found 256.1589 [M+H⁺]. ¹H NMR (300 MHz; *d₄*-CH₃OH) δ 3.37 (s, OCH₃), 4.71 (s, -CH₂-), 7.30 (m, H-Ph), 8.14 (s, H2).
(7) 6-Benzylamino-8-sulphanylpurine was prepared from 6-benzylamino-8-sulphanyl-9-(ß-D-ribofuranosyl)purine. Yield 8 mg of 6-benzylamino-8-sulphanylpurine (21%). Mp: 267-269°C. MS ESI+: 258.1 [M+H⁺]. For C₁₂H₁₁N₅S calculated 257.0735, found 258.1500 [M+H⁺]. ¹H NMR (300 MHz; *d₄*-CH₃OH) δ 4.80 (s, -CH₂-), 7.30 (m, H-Ph), 8.25 (s, H2).
(8) 6-Benzylamino-8-methylsulphanylpurine was prepared from 6-benzylamino-8-methylsulphanyl-9-(ß-D-ribofuranosyl)purine (100.8 mg, 0.250 mmol). Yield 29.7 mg of 6-benzylamino-8-methylsulphanylpurine (44%). Mp: 234-237°C. MS ESI+: 270.2 [M+H⁺]. For C₁₃H₁₃N₅S calculated 271.0892, found 272.0940 [M+H⁺]. ¹H NMR (300 MHz; *d₄*-CH₃OH) δ 2.70 (s, CH₃S), 4.80 (s, -CH₂-), 7.30 (m, H-Ph), 8.13 (s, H2).
(9) 6-(3-Methylbut-2-en-1-ylamino)-8-methylsulphanylpurine was prepared from 6-(3-methylbut-2-en-1-ylamino)-8-methylsulphanyl-9-(ß-D-ribofuranosyl)purine. Yield 13.4 mg of 6-(3-Methylbut-2-en-1-ylamino)-8-methylsulphanylpurine (33%). Mp: 96-99°C. MS ESI+: 250.2 [M+H⁺]. For C₁₁H₁₅N₅S calculated 249.1048, found 250.1060 [M+H⁺]. ¹H NMR (400 MHz; *d₄*-CH₃OH) δ 1.71 (s, -CH₃), 2.62 (s, CH₃S), 5.00 (bs, -CH₂-), 5.33 (t, *J*=5.6 Hz, -CH=), 8.10 (s, H2).
(10) 8-(3-Aminopropylamino)-6-benzylaminopurine was prepared from 8-(3-aminopropylamino)-6-benzylamino-9-(ß-D-ribofuranosyl)purine (50.3 mg, 0.117 mmol) applying this typical procedure. Yield 22 mg of 8-(3-aminopropylamino)-6-benzylaminopurine (63%). Mp: 170-173°C. MS ESI+: 298.3 [M+H⁺]. For C₅H₁₉N₇ calculated 297.1702, found 298.1950 [M+H⁺]. ¹H NMR (300 MHz; *d₄*-CH₃OH) δ 1.78 (pent., *J*=5.5 Hz, -NH-CH₂-CH₂-CH₂-NH₂), 3.00 (m, -NH-(CH₂)₂-CH₂-NH₂), 3.64 (t, *J*=5.0 Hz, -NH-CH₂-(CH₂)₂-NH₂), 4.71 (s, -CH₂-), 7.32 (m, H-Ph), 8.12 (s, H2).
(11) 6-Benzylamino-8-hydroxypurine was prepared from 6-benzylamino-8-hydroxy-9-(ß-D-ribofuranosyl)purine (19.7 mg, 0.053 mmol). Yield 8.5 mg of 6-benzylamino-8-hydroxypurine (43%). Mp: 243-247°C. MS ESI+: 242.1 [M+H⁺]. For C₁₂H₁₁N₅O calculated 241.0964, found 242.0971 [M+H⁺]. ¹H NMR (300 MHz; *d₄*-CH₃OH) δ 4.70 (s, -CH₂-), 7.30 (m, H-Ph), 8.12 (s, H2).
(12) 6-Benzylamino-8-cyanopurine was prepared from 6-benzylamino-8-cyano-9-(ß-D-ribofuranosyl)purine applying the described procedure. Yield 12.4 mg of 6-benzylamino-8-cyanopurine (50%). Mp: 210-213°C. MS ESI+: 251.2 [M+H⁺]. For C₁₃H₁₀N₆ calculated 250.0967, found 251.0980 [M+H⁺]. ¹H NMR (300 MHz; *d₆*-DMSO) δ 4.69 (s, -CH₂-), 7.25 (m, H-Ph), 8.17 (s, H2), 8.47 (bs, H9).
(13) 8-Aminomethyl-6-benzylaminopurine was prepared from 8-aminomethyl-6-benzylamino-9-(ß-D-ribofuranosyl)purine applying the typical procedure. Yield 12.4 mg of 8-aminomethyl-6-benzylamino-purine (49%). Mp: 187-189°C. MS ESI+: 254.3 [M+H⁺]. For C₁₃H₁₄N₆ calculated 254.1280, found 255.1310 [M+H⁺]. ¹H NMR (300 MHz; *d₄*-CH₃OH) δ 3.90 (s, C**H₂**NH₂), 4.71 (s, -CH₂-), 7.30 (m, H-Ph), 8.07 (s, H2).
(14) 6-Benzylamino-8-ethoxycarbonylpurine. 6-Benzylamino-8-ethoxycarbonyl-9-(ß-D-ribofuranosyl)purine (138.4 mg, 0.362 mmol) was dissolved in 2 ml 50% CF₃COOH (water solution) and 2ml ethanol. The solution was let to stand for 24 h at room temperature, then evaporated without neutralisation and the residue purified by column chromatography in CHCI₃-MeOH-NH₄OH (95:5:0.5). Yield 43.1mg of 6-benzylamino-8-ethoxycarbonylpurine (40%). Mp: 181-185°C. MS ESI-: 296.3 [M+H⁺]. For C₁₅H₁₅N₅O₂ calculated 297.1226, found 298.1162 [M+H⁺]. ¹H NMR (300 MHz; *d₆*-DMSO) δ 1.33 (t, *J*=7.0 Hz, CH₂C**H**₃), 4.39 (d, *J*=7.0 Hz, C**H₂**CH₃), 4.70 (s, -CH₂-), 7.28 (m, H-Ph), 8.26 (s, H2), 8.89 (bs, H9). IR: 1723 cm⁻¹ (C=O).
(15) 6-Benzylaminopurine-8-carboxylic acid was prepared from 6-benzylamino-9-(ß-D-ribofuranosyl)purine-8-carboxylic acid applying the typical procedure. Yield 7.1 mg of 6-benzylaminopurine-8-carboxylic acid (26%); mp: 230-232°C. MS ESI+: 270.3 [M+H⁺]. For C₁₃H₁₁N₅O₂ calculated 269.0913, found 270.1136 [M+H⁺]. ¹H NMR (300 MHz; *d₄*-CH₃OH) δ 4.70 (s, -CH₂-), 7.30 (m, H-Ph), 8.24 (s, H2).
(16) 8-Dimethylamino-6-furfurylaminopurine was prepared from 8-dimethylamino-6-furfurylamino-9-(ß-D-ribofuranosyl)purine applying the typical procedure. Yield: 70 %.White crystals, mp 245-246 °C. MS ESI+ (CV 20) m/z (rel. %): 259 [M+H]⁺ (100). ¹H-NMR (400 MHz, *d₆*-DMSO): 3.60 (d, *J*=12.1 Hz, H-5'a), 3.65 (dd, *J*=2.6 Hz, 12.1 Hz, H-5'b), 3.96 (m, H-4'), 4.13 (m, H-3'), 4.69 (m, H-2'), 4.71 (d, *J*=6.3 Hz, CH₂Fur), 5.90 (d, *J*=7.2 Hz, H-1'), 6.19 (dd, *J*=0.9 Hz, 3.2 Hz, Fur H-2), 6.34 (dd, *J*=1.8 Hz, 3.2 Hz, Fur H-3), 6.69 (bs, C⁸-NH₂, 7.23 (t, *J*=6.3 Hz, C⁶-NH), 7.52 (dd, *J*=0.9 Hz, 1.8 Hz, Fur H-4), 7.98 (s, C²-H).
(16) 8-(2-Aminoethylamino)-6-furfurylaminopurine was prepared from 8-(2-aminoethylamino)-6-furfurylamino-9-(ß-D-ribofuranosyl)purine applying the typical procedure. Yield: 88 %. Yellowish foam. MS ESI+ (CV 20) m/z (rel. %): 274 [M+H]⁺ (100). ¹H-NMR (400 MHz, *d₆*-DMSO): 3.60 (d, *J*=12.1 Hz, H-5'a), 3.65 (dd, *J*=2.6 Hz, 12.1 Hz, H-5'b), 3.96 (m, H-4'), 4.13 (m, H-3'), 4.69 (m, H-2'), 4.71 (d, *J*=6.3 Hz, CH₂Fur), 5.90 (d, *J*=7.2 Hz, H-1'), 6.19 (dd, *J*=0.9 Hz, 3.2 Hz, Fur H-2), 6.34 (dd, *J*=1.8 Hz, 3.2 Hz, Fur H-3), 6.69 (bs, C⁸-NH₂, 7.23 (t, *J*=6.3 Hz, C⁶-NH), 7.52 (dd, *J*=0.9 Hz, 1.8 Hz, Fur H-4), 7.98 (s, C²-H).
(17) 6-Furfurylamino-8-methoxypurine was prepared from 6-furfurylamino-8-methoxy-9-(ß-D-ribofuranosyl)purine applying the typical procedure. Yield: 60 %. Light beige crystals, mp 196-198 °C. MS ESI+ (CV 10) m/z (rel. %): 246 [M+H]⁺ (100). ¹H-NMR (300 MHz, *d₆*-DMSO): 4.04 (3H, s, OCH₃), 4.68 (2H, d, *J*=5.9 Hz, -HN-C**H**₂-), 6.21 (1H, d, *J*=2.4 Hz, HC³_{Fur}), 6.35 (1H, m, HC⁴_{Fur})^{a}, 6.40 (1H, bs, HC⁴_{Fur})^{b}, 7.52 (1H, d, *J*=2.4 Hz, HC⁵_{Fur}), 7.52 (1H, bs, HN⁶)^{a}, 7.55 (1H, bs, HN⁶)^{b}, 8.08 (1H, s, HC²)^{a}, 8.20 (1H, s, HC²)^{b}, 11.32 (1H, bs, HN⁷), (b = 20%), 12.49 (1H, bs, HN⁹), (a = 80%).
(18) 6-Furfurylamino-8-(2-hydroxyethyloxy)purine was prepared from 6-furfurylamino-8-(2-hydroxyethyloxy)-9-(ß-D-ribofuranosyl)purine applying the typical procedure. Yield: 31 %. White crystals, mp 182-184 °C. MS ESI+ (CV 10) m/z (rel. %): 276 [M+H]⁺ (100). ¹H-NMR (300 MHz, *d₆*-DMSO): 3.75 (2H, q, *J*=4.7 Hz, -O-CH₂C**H**₂-OH), 4.43 (2H, t, *J*=4.7 Hz, -O-C**H**₂CH₂-OH), 4.68 (2H, d, *J*=5.5 Hz, -NH-C**H**₂-), 4.97 (1H, t, *J*=5.2 Hz, -OH), 6.21 (1H, s, HC³_{Fur}), 6.35 (1H, s, HC⁴_{Fur}), 6.87 (1H, bs HN⁶)^{b}, 7.52 (1H, s, HC⁵_{Fur}), 7.54 (1H, bs, HN⁶)^{a}, 8.09 (1H, s, HC²), 11.31 (1H, bs, HN⁷), (b = 15%), 12.50 (1H, bs, HN⁹), (a = 85%).
(19) 8-Dimethylamino-6-(3-methylbut-2-en-1-ylamino)purine was prepared from 8-dimethylamino-6-(3-methylbut-2-en-1-ylamino)-9-(ß-D-ribofuranosyl)purine applying the typical procedure. Yield: 68 %. Light beige crystals, mp 246-249 °C. MS ESI+ (CV 20) m/z (rel. %): 247 [M+H]⁺ (100). ¹H-NMR (300 MHz, *d₆*-DMSO): 1.69 (6H, s, =C(CH₃)₂), 3.03 (6H, s, -N(CH₃)₂), 4.02 (2H, t, *J*=6.2 Hz, -NHC**H**₂-), 5.32 (1H, t, *J*=6.2 Hz, -CH=), 6.26 (1H, bs, HN⁶)^{b}, 6.53 (1H, bs, HN⁶)^{a}, 8.01 (1H, s, HC²), 10.68 (1H, bs, HN⁷), (b = 45%), 11.82 (1H, bs, HN⁹), (a = 55%).
(20) 8-(2-Aminoethylamino)-6-(3-methylbut-2-en-1-ylamino)purine was prepared from 8-(2-aminoethylamino)-6-(3-methylbut-2-en-1-ylamino)-9-(ß-D-ribofuranosyl)purine applying the typical procedure. Purified by column chromatography (silica gel, mobile phase chloroform/methanol/saturated aqueous ammonia 75/25/2.5). Yield: 79 %. White crystals, mp 96-102 °C. MS ESI+ (CV 20) m/z (rel. %): 262 [M+H]⁺ (100). ¹H-NMR (300 MHz, *d₆*-DMSO): 1.67 (3H, s, -CH₃), 1.81 (3H, s, -CH₃), 2.94 (2H, t, *J*=6.0 Hz, - NH-CH₂-C**H**₂-NH₂), 3.45 (2H, t, *J*=6.0 Hz, -NH-C**H**₂-CH₂-NH₂), 4.01 (2H, t, *J*=5.8 Hz, - NH-C**H**₂-CH=C), 5.31 (1H, tt, *J*=6.8 Hz, 1.5 Hz, -NH-CH₂-C**H**=C), 6.92 (1H, t, *J*=5.5 Hz, HN⁶), 7.20 (3H, bs, HN⁸,-NH₂), 7.99 (1H, s, HC²), HN⁹ - not visible.
(21) 8-(2-Aminohexylamino)-6-(3-methylbut-2-en-1-ylamino)purine was prepared from 8-(2-aminohexylamino)-6-(3-methylbut-2-en-1-ylamino)-9-(ß-D-ribofuranosyl)purine applying the typical procedure. Purified by column chromatography (silica gel, mobile phase chloroform/methanol/saturated aqueous ammonia 75/25/2.5). Yield: 68 %. Glassy off white substance. MS ESI+ (CV 25) m/z (rel. %): 318 [M+H]⁺ (100). ¹H-NMR (300 MHz, *d₆*-DMSO): 1.29 (4H, m, -NH-(CH₂)₂-(C**H**₂)₂-(CH₂)₂-NH₂), 1.55 (4H, m, -NH-(C**H**₂)₂-(CH₂)₂-(C**H**₂)₂-NH₂), 1.68 (6H, s, -CH₃), 2.65 (2H, t, *J*=6.4 Hz, -NH-(CH₂)₅-C**H**₂-NH₂), 3.26 (2H, t, *J*=5.7 Hz, -NH-C**H**₂-(CH₂)₅-NH₂), 3.99 (2H, t, *J*=6.1 Hz, -NH-C**H**₂-CH=), 5.30 (1H, t, *J*=6.9 Hz, -NH-CH₂-C**H**=C), 6.79 (1H, t, *J*=5.31 Hz, HN⁶), 7.95 (1H, s, HC²), HN⁹ - no visible.
(22) 8-Methoxy-6-(3-methylbut-2-en-1-ylamino)purine was prepared from 8-methoxy-6-(3-methylbut-2-en-1-ylamino)-9-(ß-D-ribofuranosyl)purine applying the typical procedure. Yield: 33 %. White crystals, mp 205-210 °C. MS ESI+ (CV 20) m/z (rel. %): 234 [M+H]⁺ (100). ¹H-NMR (300 MHz, *d₆*-DMSO): 1.66 (3H, s, -CH₃), 1.69 (3H, s,-CH₃), 4.03 (3H, s, -OCH₃), 4.05 (2H, t, *J*=5.7 Hz, -NH-C**H**₂-), 5.30 (1H, t, *J*=6.8 Hz, - CH=C), 6.46 (1H, bs, HN⁶)^{b}, 7.07 (1H, bs, HN⁶)^{a}, 8.07 (1H, s, HC²), 11.34 (1H, bs, HN⁷), (b = 15%), 14.40 (1H, bs, HN⁹), (a = 85%).
(23) (*E*)-8-Dimethylamino-6-(4-hydroxy-3-methylbut-2-en-1-ylamino)purine was prepared from (*E*)-8-dimethylamino-6-(4-hydroxy-3-methylbut-2-en-1-ylamino)-9-(ß-D-ribofuranosyl)purine applying the typical procedure. Yield: 84 %. White crystals, mp 245-249 °C. MS ESI+ (CV 20) m/z (rel. %): 263 [M+H]⁺ (100). ¹H-NMR (300 MHz, *d₆*-DMSO): 1.65 (3H, s, =C-CH₃), 3.03 (6H, s, -N(CH₃)₂), 3.81 (2H, s, -C**H**₂-OH), 4.09 (2H, t, *J*=6.1 Hz, -NH-C**H**₂), 4.77 (1H, bs, -OH), 5.54 (1H, tt, *J*=6.8 Hz, 1.5 Hz, -CH₂-C**H**=), 6.48 (1H, bs, HN⁶), 8.01 (1H, s, HC²), 10.79 (1H, bs, HN⁷), (b = 52%), 11.76 (1H, bs, HN⁹), (a = 48%).

**Example 8.** Table 1 lists additional compounds prepared by the various methods described above.

**Table 1: More Examples of Compounds of this Invention**

| **No.** | **Substituent** | | **CHN analyses calculated / found** | | | **ESI-MS [M+H+]** |
|---|---|---|---|---|---|---|
| | **R6** | **R8** | **%C** | **%H** | **%N** | |
| **1** | furfurylamino | Br | 40.8/40.0 | 2.7/2.4 | 23.8/22.9 | 295 |
| **2** | benzylamino | Br | 47.4/47.1 | 3.3/3.0 | 23.0/22.3 | 304 |
| **3** | (Z)-(4-hydroxy-3-methylbut-2-en-1-yl) amino | Br | 40.3/39.8 | 4.1/4.0 | 23.5/23.0 | 299 |
| **4** | (3-methylbut-2-en-1-yl)amino | Br | 42.6/41.9 | 4.3/3.9 | 24.8/24.5 | 283 |
| **12** | 2-methoxylbenzylamino | Br | 46.7/46.5 | 3.6/3.7 | 20.9/21.5 | 335 |
| **13** | 3-methoxylbenzylamino | Br | 46.7/46.0 | 3.6/3.3 | 20.9/20.0 | 335 |
| **14** | 4-methoxylbenzylamino | Br | 46.7/46.2 | 3.6/3.3 | 20.9/20.5 | 335 |
| **17** | 2.3-dihydroxybenzylamino | Br | 42.9/42.4 | 3.0/3.2 | 20.8/20.2 | 337 |
| **18** | 2-hydroxy-3-methoxybenzylamino | Br | 44.6/44.3 | 3.5/3.4 | 20.0/19.6 | 351 |
| **19** | 3.4-dimethoxybenzylamino | Br | 46.2/46.3 | 3.9/3.0 | 21.9/21.2 | 365 |
| **61** | furfurylamino | (CH₃)₂N | 55.8/55.5 | 5.5/5.5 | 32.5/32.4 | 259 |
| **62** | benzylamino | (CH₃)₂N | 62.7/62.4 | 6.0/6.1 | 31.3/30.7 | 269 |
| **63** | (Z)-(4-hydroxy-3-methylbut-2-en-1-yl) amino | (CH₃)₂N | z4.7 | 6.9/6.9 | 32.0/31.4 | 263 |
| **71** | 2-methoxylbenzylamino | (CH₃)₂N | 60.4/59.9 | 6.1/6.3 | 28.2/27.7 | 299 |
| **72** | 3-methoxylbenzylamino | (CH₃)₂N | 60.4/60.0 | 6.1/6.0 | 28.2/27.9 | 299 |
| **74** | 2-hydroxy-3-methoxybenzylamino | (CH₃)₂N | 57.3/56.9 | 5.8/5.7 | 26.7/26.1 | 315 |
| **75** | 3,4-dimethoxybenzylamino | (CH₃)₂N | 58.5/58.0 | 6.1/6.1 | 25.6/25.2 | 329 |
| **82** | furfurylamino | NH₂ | 52.2/52.0 | 4.4/4.0 | 36.5/36.8 | 231 |
| **83** | benzylamino | NH₂ | 60.0/59.5 | 6.0/5.9 | 34.0/34.6 | 241 |
| **84** | (Z)-(4-hydroxy-3-methylbut-2-en-1-yl) amino | NH₂ | 51.3/50.9 | 6.0/5.9 | 35.9/35.5 | 235 |
| **85** | (E)-(4-hydroxy-3-methylbut-2-en-1-yl) amino | NH₂ | 51.3/51.0 | 6.0/5.9 | 35.9/35.6 | 235 |
| **87** | (3-methylbut-2-en-1-yl)amino | NH₂ | 55.0/54.3 | 6.5/6.4 | 38.5/39.3 | 219 |
| **97** | 2-methoxylbenzylamino | NH₂ | 57.8/57.0 | 5.2/5.0 | 31.1/30.6 | 271 |
| **98** | 3-methoxylbenzylamino | NH₂ | 57.8/57.3 | 5.2/5.2 | 31.1/30.5 | 271 |
| **99** | 4-methoxylbenzylamino | NH₂ | 57.8/57.2 | 5.2/5.2 | 31.1/30.9 | 271 |
| **102** | 2,3-dihydroxybenzylamino | NH₂ | 52.9/52.3 | 4.4/4.4 | 30.9/30.3 | 273 |
| **103** | 3,4-dihydroxybenzylamino | NH₂ | 52.9/52.5 | 4.4/4.4 | 30.9/30.4 | 273 |
| **104** | 2-hydroxy-3-methoxybenzylamino | NH₂ | 54.5/53.8 | 4.9/4.8 | 29.4/29.5 | 287 |
| **105** | 3-hydroxy-4-methoxybenzylamino | NH₂ | 54.5/54.0 | 4.9/4.9 | 29.4/29.1 | 287 |
| **106** | 3,4-dimethoxybenzylamino | NH₂ | 56.0/55.9 | 5.4/5.2 | 28.0/27.3 | 301 |
| **120** | furfurylamino | CH₃O | 53.9/53.3 | 4.5/4.4 | 28.6/28.1 | 246 |
| **121** | benzylamino | CH₃O | 61.2/60.9 | 5.1/5.0 | 27.4/26.9 | 256 |
| **122** | (Z)-(4-hydroxy-3-methylbut-2-en-1-yl) amino | CH₃O | 53.0/52.7 | 6.1/6.0 | 28.1/28.7 | 250 |
| **123** | (3-methylbut-2-en-1-yl)amino | CH₃O | 56.6/56.0 | 6.5/5.9 | 30.0/27.3 | 234 |
| **127** | 2-methoxylbenzylamino | CH₃O | 58.9/59.4 | 5.3/5.4 | 24.5/24.0 | 286 |
| **128** | 3-methoxylbenzylamino | CH₃O | 58.9/58.4 | 5.3/5.3 | 24.5/23.9 | 286 |
| **130** | 2-hydroxy-3-methoxybenzylamino | CH₃O | 55.8/55.2 | 5.0/4.8 | 23.2/23.0 | 302 |
| **131** | 3,4-dimethoxybenzylamino | CH₃O | 57.1/56.6 | 5.4/5.3 | 22.2/21.8 | 316 |
| **187** | furfurylamino | CH₃OCO | 52.7/52.1 | 4.0/3.8 | 25.6/25.1 | 274 |
| **188** | benzylamino | CH₃OCO | 59.4/59.0 | 4.6/4.4 | 24.7/24.8 | 284 |
| **190** | 3-methoxylbenzylamino | CH₃OCO | 57.5/57.2 | 4.8/4.9 | 22.3/22.3 | 314 |
| **194** | furfurylamino | CH₃CH₂OCO | 54.4/53.9 | 4.6/4.5 | 24.3/23.9 | 288 |
| **195** | benzylamino | CH₃CH₂OCO | 60.6/60.0 | 5.1/5.0 | 23.55 | 298 |
| **197** | 3-methoxylbenzylamino | CH₃CH₂OCO | 58.7/58.3 | 5.2/5.1 | 21.4/21.0 | 328 |
| **208** | furfurylamino | NH₂(CH₂)₃NH | 54.3/53.7 | 6.0/5.7 | 34.1/33.6 | 288 |
| **209** | benzylamino | NH₂(CH₂)₃NH | 60.6/60.0 | 6.4/6.2 | 33.0/32.6 | 298 |
| **210** | (Z)-(4-hydroxy-3-methylbut-2-en-1-yl) amino | NH₂(CH₂)₃NH | 53.6/52.9 | 7.3/7.1 | 33.6/33.1 | 292 |
| **211** | (3-methylbut-2-en-1-yl)amino | NH₂(CH₂)₃NH | 56.7/56.2 | 7.7/7.8 | 35.6/35.2 | 276 |

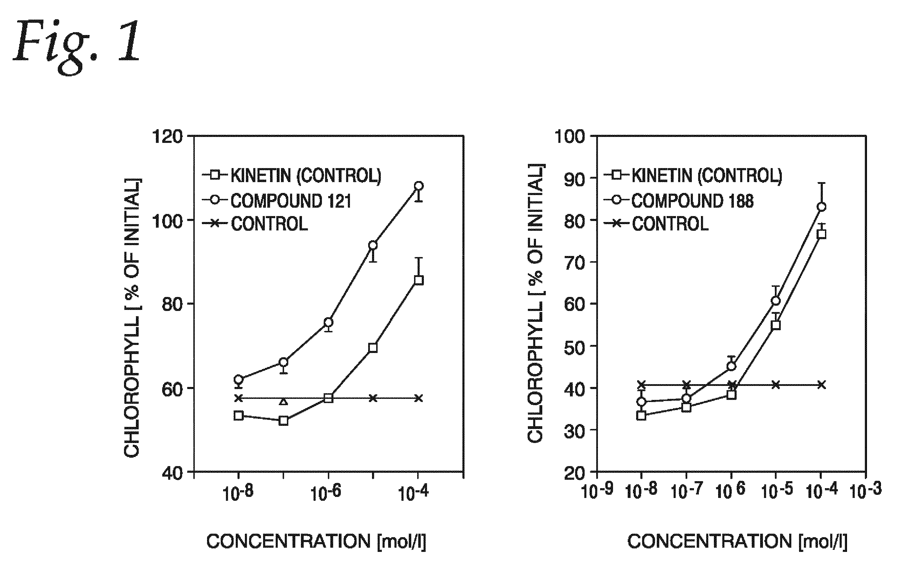

**EXAMPLE 9: Senescence Inhibition by Novel Compounds Tested on Winter Wheat Leaf Segments.** Seeds of winter wheat, *Triticum aestivum* cv. Hereward, were washed under running water for 24 hours and then sown on vermiculite soaked with Knop's solution. They were placed in the grown chamber at 25C with a 16/8 hours light period at 50 mmol.m⁻².s⁻¹. After 7 days, the first leaf was fully developed and the second leaf had started to grow. A tip section of the first leaf, approximately 35 mm long, was removed from 5 seedlings and trimmed slightly to a combined weight of 100 mg. The basal ends of the five leaf tips were placed in the wells of a microtiter polystyrene plate containing 150 ml of a cytokinin solution each. The entire plate was inserted into a plastic box lined with paper tissues soaked in distilled water to prevent leaf sections from drying out. After 96 hours incubation in the dark at 25C, the leaves were removed and chlorophyll extracted by heating at 80°C for 10 min in 5 ml of 80% ethanol (v/v). The sample volume was then restored to 5 ml by the addition of 80% ethanol (v/v). The absorbance of the extract was recorded at 665 nm. In addition, chlorophyll extracts from fresh leaves and leaf tips incubated in deionized water were measured. The set up of the assay is shown on Figure 1. From the obtained data, the concentration with highest activity was selected for each compound tested. Relative activity of the compound at this concentration was calculated (see Table 2 below). The activity obtained for 10⁻⁴ M kinetin (Kin) was set as 100%. The values shown are means of five replicates and the whole experiment was repeated twice. The compounds to be tested were dissolved in dimethylsulfoxide (DMSO) and the solution brought up to 10⁻³ M with distilled water. This stock was further diluted in distilled water to concentrations ranging from 10⁻⁸ M to 10⁻⁴ M. The final concentration of DMSO did not exceed 0.2% and therefore did not affect biological activity in the assay system used.

As summarized in Table 2 below, the presence of C-8 substitutent was beneficial and led to an increase of antisenescent properties (also see Figure 1).

**Table 2: Effect on retention of chlorophyll in excised wheat leaf tips.**

| **Compound*** | **Concentration with highest activity (mol/l)** | **Activity (%)** |
|---|---|---|
| **kinetin** | 10⁻⁴ | 100 |
| **2** | 10⁻⁴ | 144.76 (± 6) |
| **62** | 10⁻⁴ | 106.13 (± 1) |
| **195** | 10⁻⁴ | 122.06 (± 2) |

| | | |
|---|---|---|
| * Activity of kinetin set at 100%; standard deviations are of the mean for 10 replicate determinations; numbers identifying test compounds are from Table 1. | | |

**EXAMPLE 10: Testing of novel compounds in Amaranthus bioassay.** Standard Amaranthus bioassay was performed with several modifications. The seeds of Amaranthus caudatus var. atropurpurea were surface-sterilized in 10% N-chlorobenzene sulfonamide (w/v) for 10 min and washed 5 times in deionized water. They were placed in 14 cm Petri dishes containing paper tissues saturated with deionized water. After 72 hours of cultivation at 25°C in darkness, the roots of the seedlings were cut off. The explants, consisting of two cotyledons and hypocotyls, were placed in 5 cm Petri dishes on two layers of filter paper soaked in 1 ml of incubation medium containing 10 µmol Na₂HPO₄-KH₂PO₄, pH 6.8, 5 µmol tyrosine and the cytokinin to be tested. There were 20 explants per dish. The procedure was carried out under a green safe light in a darkroom. After a 48 hours of incubation at 25°C in darkness, betacyanin was extracted by freezing the explants in 4 ml 3.33 µM acetic acid. The concentration of betacyanin was determined by comparing the absorbance at 537nm and 620nm as follows: DA = A₅₃₇ₙₘ - A₆₂₀ₙₘ. From the obtained data, the concentration with highest activity was selected for each compound tested. Relative activity of the compound at this concentration was calculated (see Table 3 below). The activity obtained for 10⁻⁵ M kinetin (Kin) was set as 100%. The values shown in Table 3 are means of five replicates and the entire test was repeated twice.

The cytokinins to be tested were dissolved in dimethylsulfoxide (DMSO) and the solution brought up to 10⁻³ M with distilled water. This stock was further diluted in the respective media used for the biotest to concentrations ranging from 10⁻⁸ M to 10⁻⁴ M. The final concentration of DMSO did not exceed 0.2% and therefore did not affect biological activity in the assay system used.

**Table 3: The effect on betacyanin content in Amaranthus caudatus cotyledon/hypocotyl explants.**

| **Compound** | **Concentration with highest activity (mol/l)** | **Activity (%)** |
|---|---|---|
| **kinetin*** | 10⁻⁵ | 100 |
| **82** | 10⁻⁴ | 147.45 (± 11) |
| **87** | 10⁻⁴ | 171.36 (± 10) |
| **121** | 10⁻⁴ | 121.15 (± 3) |
| **188** | 10⁻⁴ | 122.51 (± 5) |
| **195** | 10⁻⁴ | 122.55 (± 13) |

| | | |
|---|---|---|
| * Activity of kinetin set at 100%. | | |

**EXAMPLE 11: The effect on plant cell division.** Cytokinin-dependent tobacco callus *Nicotiana tabacarm* L. cv. Wisconsin 38 was maintained at 25C in darkness on modified MS medium, containing 4µmol/l nicotinic acid, 2.4 µmol/l pyridoxine hydrochloride, 1.2 µmol/l thiamine, 26.6 µmol/l glycine, 1.37 µmol/l glutamine, 1.8 µmol/l myo-inositol, 30g/l of sucrose, 8g/l of agar, 5.37 µmol/l NAA and 0.5 µmol/l BAP. Subcultivation was carried out every three weeks. Fourteen days before the bioassay, the callus tissue was transferred to the media without BAP. Biological activity was determined from the increase in fresh callus weight after four weeks of cultivation. Five replicates were prepared for each cytokinin concentration and the entire test was repeated twice. From the obtained data, the concentration with highest activity was selected for each compound tested. Relative activity at this concentration was calculated (see Table 4 below). The activity obtained for 10⁻⁶ M kinetin (Kin) was set as 100%.

The cytokinins to be tested were dissolved in dimethylsulfoxide (DMSO) and the solution brought up to 10⁻³M with distilled water. This stock was further diluted in the respective media used for the biotest to concentrations ranging from 10⁻⁸ M to 10⁻⁴ M. The final concentration of DMSO did not exceed 0.2% and therefore did not affect biological activity in the assay system used.

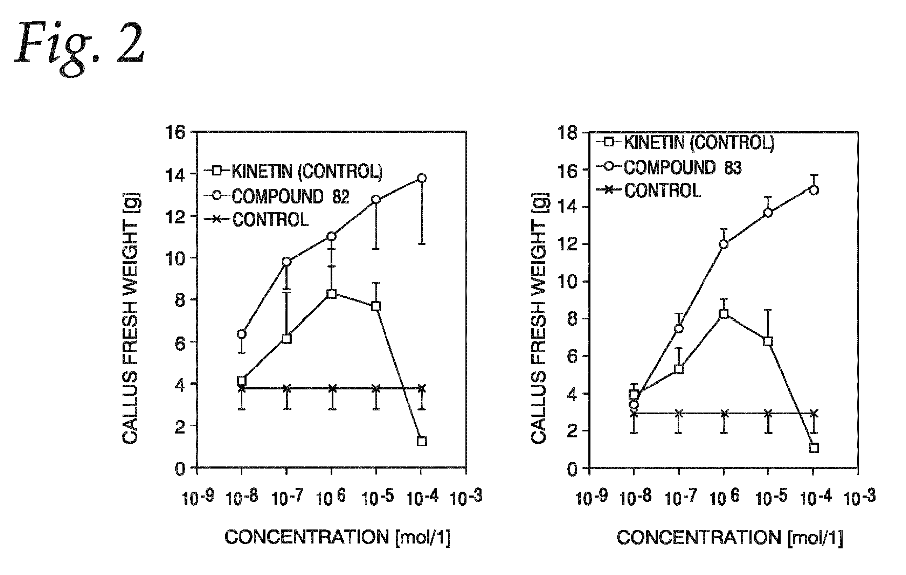

As summarized in Table 4, the presence of the C-8 substitutents shifted the optimal concentration to higher concentrations. However, in contrast to kinetin, which shows sharp concentration optimum, most of the new compounds have markedly extended range of the optimal concentration (over two to three orders of magnitude). As shown in Figure 2, C-8 derivatives of cytokinins thus loose their cytotoxic effects when applied in higher concentrations.

**Table 4. The effect on growth of cytokinin-dependent tobacco callus Nicotiana tabacum L. cv. Wisconsins 38.**

| **Compound** | **Concentration with highest activity (mol/l)** | **Activity (%)]** |
|---|---|---|
| **kinetin*** | 10⁻⁵ | 100 |
| **2** | 10⁻⁴ | 174.83 (± 11) |
| **62** | 10⁻⁴ | 165.64 (± 10) |
| **82** | 10⁻⁴ | 150.10 (± 8) |
| **83** | 10⁻⁴ | 197.08 (± 15) |
| **87** | 10⁻⁵ | 150.44 (± 9) |
| **121** | 10⁻⁵ | 129.11 (± 5) |
| **188** | 10⁻⁶ | 148,91 (± 8) |
| **209** | 10⁻⁴ | 150,01 (± 11) |

| | | |
|---|---|---|
| * Activity of kinetin set at 100%. | | |

**EXAMPLE 12: *In vitro* Cytotoxic Activity (Metabolisation of Calcein AM).** Because toxic compounds negatively influence metabolic processes of cells, many standard cytotoxicity assays are based on measurement of metabolisation rate of various artificial substrates. Resulting product is then quantified, for example, by means of spectrometry. The assays can be easily modified for use in 96-well plates. For evaluation of cytotoxicity of the 6,8-disubstituted purines of this invention, a microtiter assay based on quantification of metabolisation of Calcein AM was used. The assay is widely used in drug screening programs and in chemosensitivity testing. In live cells, Calcein AM is enzymatically hydrolysed and acummulation of resulting calcein is manifested by green fluorescence.

The following human cell lines were used for rutine screening of the compounds: normal diploid fibroblasts BJ, T-lymphoblastic leukemia cell line CEM, promyelocytic leukemia cell line HL-60, erytroid leukemia cell line K-562, breast carcinoma cell line MCF-7, osteosarcoma cell line HOS and melanoma cell line G-361. The cells were maintained in Nunc/Corning 80 cm² plastic tissue culture flasks and cultured in cell culture medium (DMEM with 5 g/l glucose, 2mM glutamine, 100 U/ml penicillin, 100 mmg/ml streptomycin, 10% fetal calf serum and sodium bicarbonate).

The cell suspensions were prepared and diluted according to the particular cell type and the expected target cell density (2.500-30.000 cells per well based on cell growth characteristics) and pipetted (80 ml) into 96-well plates. Inoculates were allowed a preincubation period of 24 hours at 37C and 5% CO₂ for stabilization. Tested compound was added in total volume of 20 ml of water at time zero. Usually, test compound was evaluated at six 3-fold dilutions. In routine testing, the highest concentration tested was 100 mM, but it could have been adjusted because of limited solubility of a compound. All drug concentrations were tested in triplicates.

Incubations of cells with the test compounds lasted for 72 hours at 37C, in 5% CO₂ atmosphere and 100% humidity. At the end of incubation period Calcein AM in PBS was added into final concentration of 1 µg/ml. After another 1 hour of incubation fluorescence (FD) was measured with the Labsystem FIA Reader Fluoroscan Ascent (UK). Growth inhibition (GI) was estimated using the following equitation: GI = (mean FD_{drug exposed wells} - mean FD_{blank}) / (mean FD_{control wells}- mean FD_{blank}) x 100%. The GI₅₀ value, the drug concentration causing 50% reduction of Calcein AM conversion, was calculated from the obtained dose response curves.

Cytoxicity of compounds was tested on panel of cell lines of different histogenetic and species origin. As shown in Table 5, with one exception (compound 82, cell line K-562, GI₅₀ = 68.2), GI₅₀ of 6,8-disubstituted purines exceeded maximal concentration tested which suggests that the compounds could be applied at concentrations causing desired effect without negative side effects.

**Table 5. Cytotoxicity for Different Cancer Cell Lines**

| **Compound** | **Cell line tested / IC₅₀ (µmol/L)** | | | | | | **Highest concentration tested (µmol/L)** |
|---|---|---|---|---|---|---|---|
| | **BJ** | **CEM** | **K-562** | **MCF7** | **HOS** | **G-361** | |
| **2** | >50 | >50 | >50 | >50 | >50 | >50 | 50 |
| **3** | >50 | >50 | >50 | >50 | >50 | >50 | 50 |
| **63** | >50 | >50 | >50 | >50 | >50 | >50 | 50 |
| **83** | >50 | >50 | >50 | >50 | >50 | >50 | 50 |
| **84** | >50 | >50 | >50 | >50 | >50 | >50 | 50 |
| **85** | >50 | >50 | >50 | >50 | >50 | >50 | 50 |
| **87** | >50 | >50 | >50 | >50 | >50 | >50 | 50 |
| **97** | >50 | >50 | >50 | >50 | >50 | >50 | 50 |
| **98** | >50 | >50 | >50 | >50 | >50 | >50 | 50 |
| **99** | >50 | >50 | >50 | >50 | >50 | >50 | 50 |
| **102** | >50 | >50 | >50 | >50 | >50 | >50 | 50 |
| **103** | >50 | >50 | >50 | >50 | >50 | >50 | 50 |
| **104** | >50 | >50 | >50 | >50 | >50 | >50 | 50 |
| **105** | >50 | >50 | >50 | >50 | >50 | >50 | 50 |
| **120** | >50 | >50 | >50 | >50 | >50 | >50 | 50 |
| **121** | >50 | >50 | >50 | >50 | >50 | >50 | 50 |
| **122** | >50 | >50 | >50 | >50 | >50 | >50 | 50 |
| **130** | >50 | >50 | >50 | >50 | >50 | >50 | 50 |
| **187** | >50 | >50 | >50 | >50 | >50 | >50 | 50 |
| **194** | >50 | >50 | >50 | >50 | >50 | >50 | 50 |
| **208** | >50 | >50 | >50 | >50 | >50 | >50 | 50 |
| **209** | >50 | >50 | >50 | >50 | >50 | >50 | 50 |
| **210** | >50 | >50 | >50 | >50 | >50 | >50 | 50 |
| **211** | >50 | >50 | >50 | >50 | >50 | >50 | 50 |

**EXAMPLE 13: *In vitro* Cytotoxic Activity (metabolisation of MTT).** MTT (metabolic tetrazolium toxicity) assay is a standard colorimetric assay for evaluation of cytotoxicity. Mitochondrial dehydrogenase activity converts yellow MTT into violet formazan which is measured by means of spectrometry.

Human diploid fibroblast BJ (passage 18-22) were seeded into 96-well plate (5.000 cells per well). After 6 hours cultivation medium (DMEM with 5 g/l glucose, 2mM glutamine, 100 U/ml penicillin, 100 mmg/ml streptomycin, 10% fetal calf serum and sodium bicarbonate) was replaced with the cultivation medium containing test compounds in concentration range of 0-200 mM. Highest concentration was adjusted if the solubility of the compound was limiting. Every concentration was tested in pentaplicate. MTT was added to the cells after 72 hours incubation (final concentration 0.5 mg / ml) and incubation continued for another 3 hours. MTT was solubilised by DMSO and absorbance at 570 nm was measured. Growth inhibition (GI) was estimated using the following equitation: GI = (mean A drug exposed wells- mean A_{blank} / mean A _{control wells}-mean A_{blank}) x 100%. The GI₂₀ value, the drug concentration causing 20% decrease in mitochondrial dehydrogenase activity, was calculated from the obtained dose response curves.

As shown in Table 6, GI₂₀ of 6,8-disubstituted purines exceded maximal concentration tested which suggests that the compounds could be applied at concentrations causing desired effect without negative side effects.

**Table 6. Cytotoxicity for Human Diploid Fibroblasts**

| **Compound** | **GI₂₀ (µmol/L)** | **Maximum tested concentration (µmol/L)** |
|---|---|---|
| **2** | >50 | 50 |
| **63** | >100 | 100 |
| **83** | >100 | 100 |
| **84** | >100 | 100 |
| **85** | >100 | 100 |
| **87** | >100 | 100 |
| **97** | >100 | 100 |
| **98** | >100 | 100 |
| **99** | >100 | 100 |
| **102** | >100 | 100 |
| **103** | >100 | 100 |
| **104** | >100 | 100 |
| **105** | >100 | 100 |
| **209** | >100 | 100 |
| **210** | >100 | 100 |
| **211** | >100 | 100 |

### EXAMPLE 14: Biological Effects on Human Skin Fibroblasts.

A short-term treatment of human skin fibroblasts with novel test compounds has been carried out. A variety of biological characteristics of such human skin fibroblasts were examined in order to evaluate such test compounds for cosmetic and/or anti-ageing applications. These characteristics included:
A) Growth characteristics:
   (1) Cell attachment frequency: to rule out immediate toxicity;
   (2) Cell survival: to rule out delayed toxicity;
   (3) Growth rates: to determine cell division stimulatory/inhibitory potential;
B) Cellular parameters:
   (4) Cellular morphology: to evaluate cytoplasmic and nuclear stability;
   (5) Cytoskeleton organization: to evaluate the stability of the cytoskeleton;
   (6) Appearance of senescence-specific β-galactosidase: to rule out pro-aging effects;
   (7) Lysosomal staining (Neutral red);
   (8) JC-1 Staining of Mitochondria: for evaluation of mitochondrial function;
   (9) Rejuvenation studies: for evaluation of reversion effect from aged phenotype to young phenotype; and
C) Biochemical approaches:
   (10) Proteasomal activities: to determine cellular capacity to degrade abnormal proteins.

**Experimental Procedures.** A stock solution of 4mM for 6-furfurylamino-8-aminopurine and 8mM for other compounds were prepared by dissolving about 30 mg per ml 1N HCl, followed by its appropriate dilution by the addition of Hank's buffer. The stock solution was filter sterilized, stored at 4°C, and was used for experiments by dilution it in the cell culture medium as required.

All cell culture experiments were performed on early and mid passage cultures (about 20 to 40% lifespan completed) of normal human skin fibroblast line, designated ASF-2. In order to check the effects of test compounds on senescent cells, late passage cells with more than 90% lifespan completed were used. ASF-2 cell line was established from a mammary skin biopsy obtained from a young, non-smoking and healthy female at the time of breast reduction operation. Normal culture conditions of medium (DMEM) containing antibiotics, 10% foetal calf serum, and incubation at 37°C with 95% humidity were used. The effects of the test compounds were determined using the following procedures. Growth characteristics. Short-term growth experiments were performed using 24-well tissue culture plates (growth area 1.9 cm²). Freshly prepared cell suspensions from mass cultures of ASF-2 cells maintained in our labs were used. About 10,000 cells were seeded into 6 sets of 24-well plates. The cells were allowed to attach and stabilize for 24hr in normal culture medium to achieve various final concentrations (range 40 to 500µM). Culture medium was changed with the addition of test chemicals twice a week. The numbers of cells were counted after different days of treatment in 2 wells from each concentration of the test chemical, by following the normal method of cell trypsinization and counting using a Coultercounter. The third well in each category was fixed by cold methanol and stained with Giemsa stain for permanent record and for photography. The experiment was carried on for until the cultures became fully confluent and no further growth was possible.

Lysosomal activity. Neutral Red is preferentially taken up into the lysosomes of the cell. Fibroblast cells are maintained in culture and exposed to test compounds over a range of concentrations. The cultures are visually examined after 72 hours, and the number of viable cells and/or the total cell protein content determined, after 72 hours exposure, by the Neutral Red Uptake method. Advantage of Neutral Red assay is that it detects only viable cells. Any material having a localized effect upon the lysosomes will, therefore, result in an artificially low (or possibly high) reflection of cell viability and cell number. This factor does, however, make the system useful to detect those compounds which selectively affect the lysosomes, especially when it is used in conjunction with other tests capable of determining cell number.

Cell survival, toxicity, and JC-1 staining. Cell survival after exposure to various doses of the test compounds was measured with the 3-(4, 5 dimethylthiazol-2-yl)-2, 5-diphenyltetrazolium bromide (MTT) assay. About 5,000 cells were seeded per well in a 96-well plate 24 h before the experiment. Cells were then treated with various doses of the compound to be tested. The wells were washed in Hank's and new medium was added. After three days, MTT (Sigma, M2128) was added at 0.5 mg/ml in medium. After 4 h, MTT was collected and then dissolved in isopropanol and HCl over a 12-16 h period. The absorbance at 595 nm was measured.

JC-1 is a cationic dye which exhibits a potential-dependant accumulation in mitochondria and is indicated by a fluorescence emission shift from green (~525 nm) to red(~590 nm) and is used as an indicator of mitochondrial potential after three days of treatment with the test compound on dermal fibroblasts. Briefly, stock solution of JC-1 (Sigma, M2128) was made at 1mg/ml in dimethyl sulfoxide. Fresh staining solution (10 µg/ml) was prepared by diluting the stock solution in warm (37°C) culture medium supplemented with 10% calf serum (final conc. 1 µg/ml). Around 3000 cells/ml were seeded in 4 chambered flasks. After three days of incubation, medium is aspirated and one ml of JC-1 staining medium is added, after incubation for 10 mins a cover slip is quickly added and viewed under fluorescent microscope at excitation wavelengths of 488 and 647 nm. Pictures are taken for records; polarized mitochondria are marked by punctate orange-red fluorescent staining. On depolarization, the orange red punctate staining is replaced by diffuse green monomer fluorescence.

Actin staining. The pattern of cytoskeletal actin staining was studied by staining the cells with florescent ligand FITC-labelled Phalloidin, using a florescence microscope. Proteasomal activity. Chymotrypsin-like activity in cell extracts prepared from treated and untreated cells was determined by *in vitro* degradation of oligopeptides by the proteasome.

### Results.

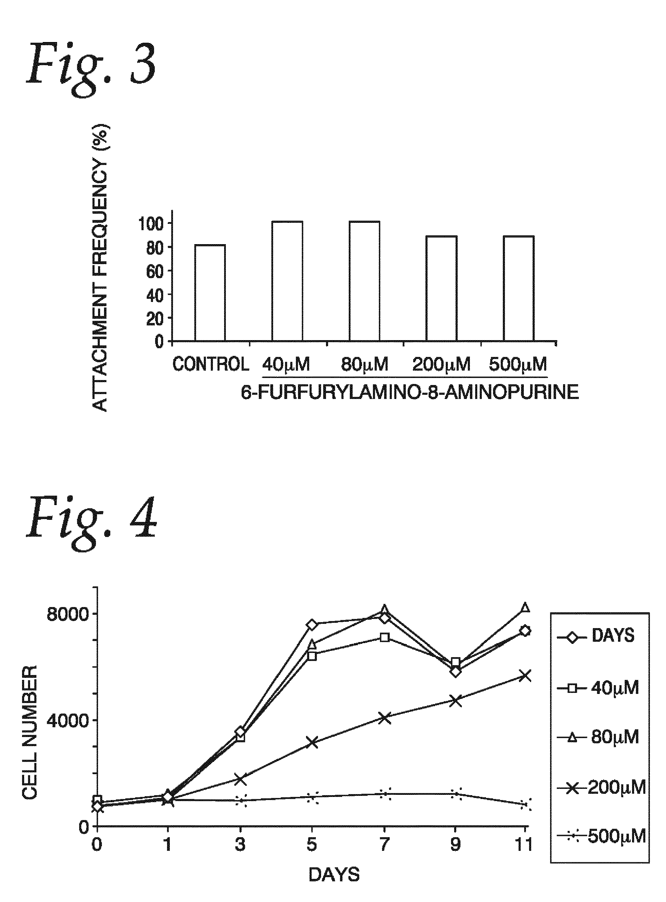

Cell attachment. Percent of cells attached to the surface of the culture flask was not affected to any significant extent after six hours of treatment with 40 to 500 µM 6-furfurylamino-8-aminopurine. Figure 3 shows that 6-furfurylamino-8-aminopurine is not immediately toxic for human skin fibroblasts. Therefore, for all further experiments, 6-furfurylamino-8-aminopurine was added to the culture medium at the time of seeding the cells.

Short-term growth. The effects of 8-amino kinetin treatment on short-term growth are shown in Figure 4. Cell growth was similar in untreated and 80 µM samples; at higher concentrations (200 to 500 µM), cellular growth was significantly inhibited and after 9 days there was almost a total inhibition. Since beta-galactosidase staining did not show any increase in the number of prematurely senescent cells, it appears that concentrations up to 80 µM of 6-furfurylamino-8-aminopurine may be suitable for long term treatment of human skin fibroblasts.

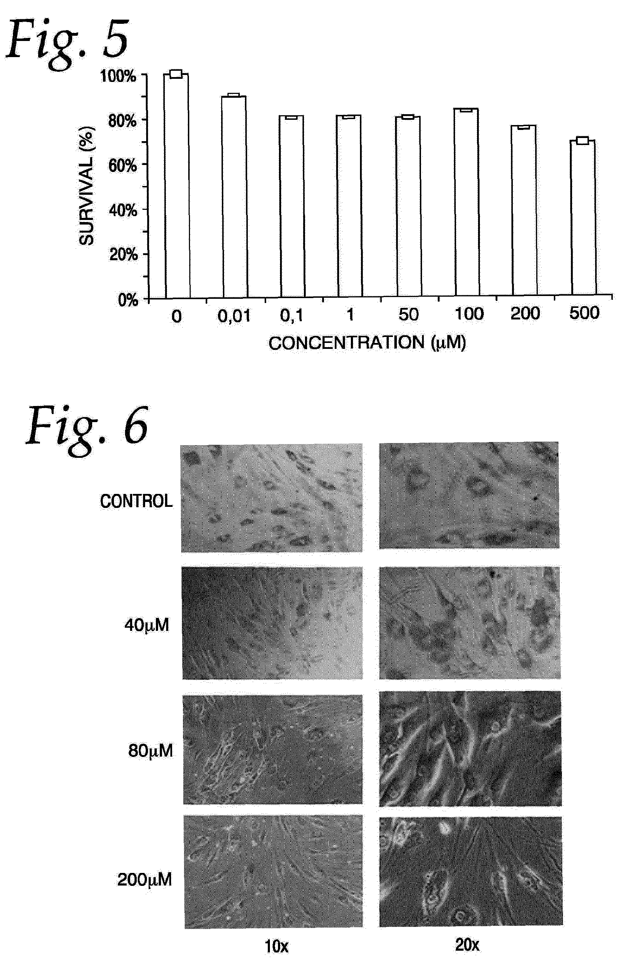

Cell survival and toxicity. Mitochondrial dehydrogenase from living cells is able to convert soluble MTT to an insoluble formazen via a reduction reaction. A wide range of concentrations of inventive compound **82** (ranging from 0.01-500 µM) on young fibroblasts were tested for determining the cell survival effects on 8 amino kinetin. We show by MTT assay that treatment on cells shows little toxic effects dermal fibroblasts until a dose of 100 µM. Figure 5 shows that there was 15 % reduction in survival on inventive compound **82** treated cells at 100 µM. However there was a reduction in cell survival at a higher dose (500 µM).

Lysosomal Staining. As shown in Figure 6, lysosomes were larger and more numerous in **82** treated cells compared to the controls. The results suggest there is an increase in the lysosomal enzyme activity and staining intensity.

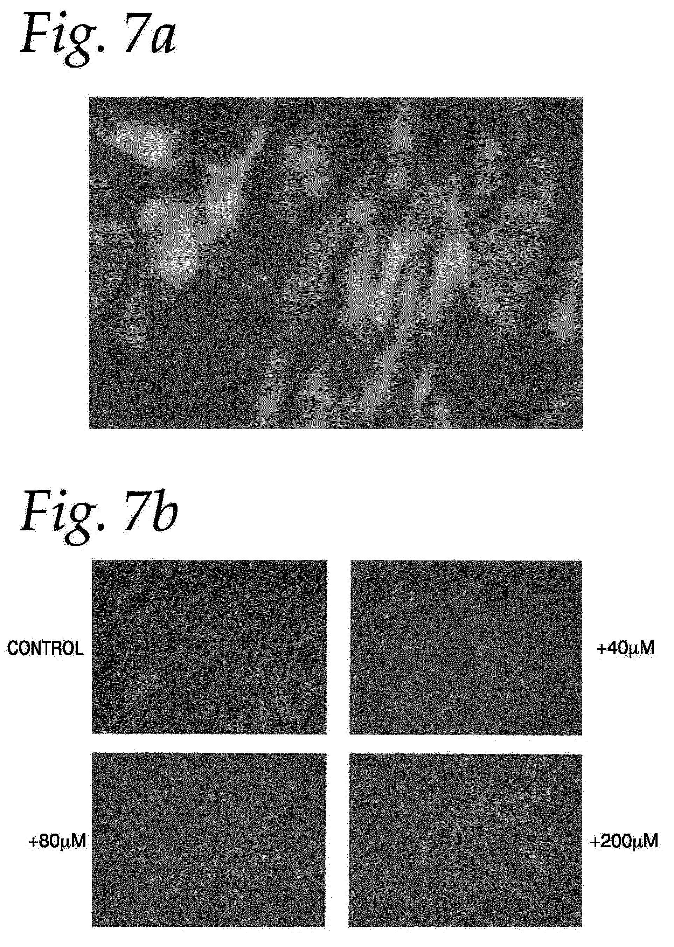

Dermal fibroblasts on **82** pre-treatment for 3 days were stained with JC-1. Fig 7 [A&B] shows two populations of cells. Polarized mitochondria from live cells [A] are marked by punctate orange-red fluorescent staining by the formation and maintenance of J-aggregates. On the other hand, cells [B] when treated with staurosporine (negative control) drastically reduced the uptake of JC-1and J-aggregate formation. Inventive compound **82** treatment on cells show punctate orange-red fluorescent staining.

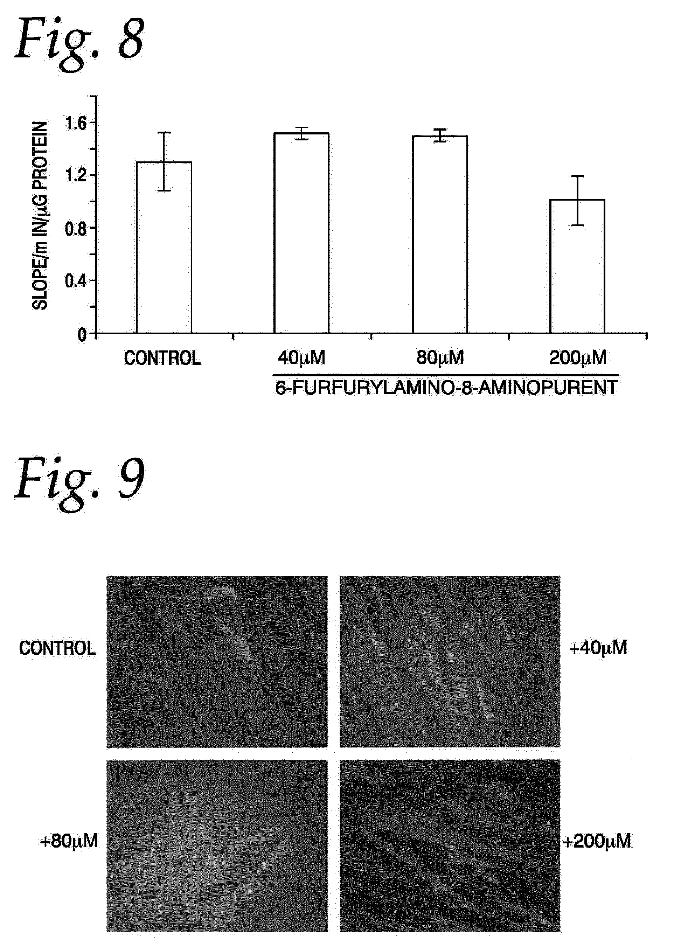

Proteasomal activity. The effects of compound **82** on proteasomal activity is shown in the Figure 8. Treatment (40 µM - 200 µM) with **82** on dermal fibroblasts showed no inhibition when compared to those of untreated on the proteasomal activity of human cells using one of the three types of proteasomal activities, namely chymotrypsin-like. 5-10% improvement in the activity was observed.

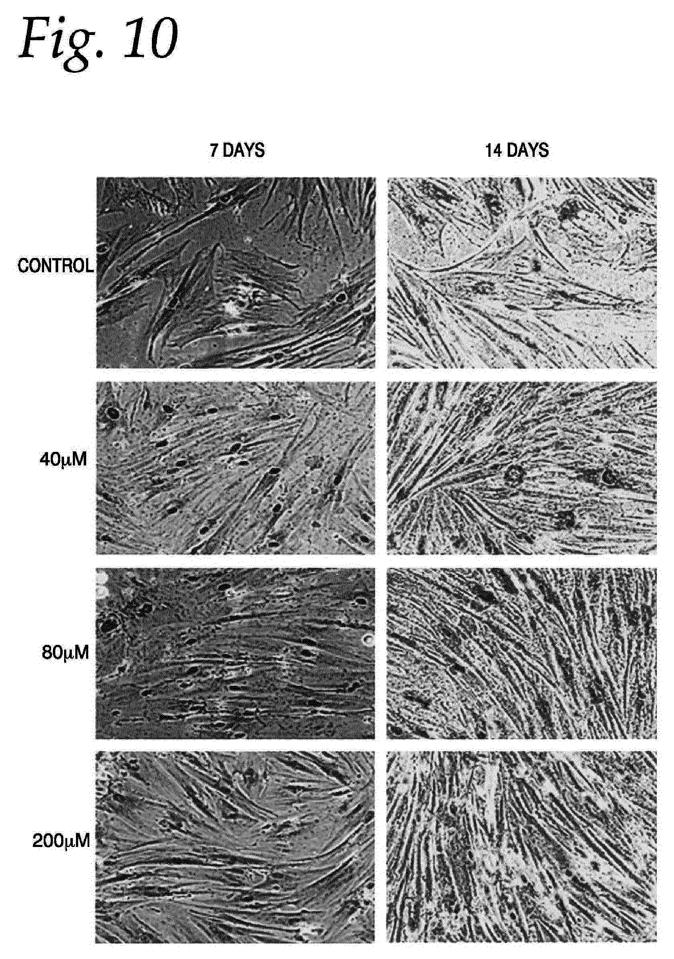

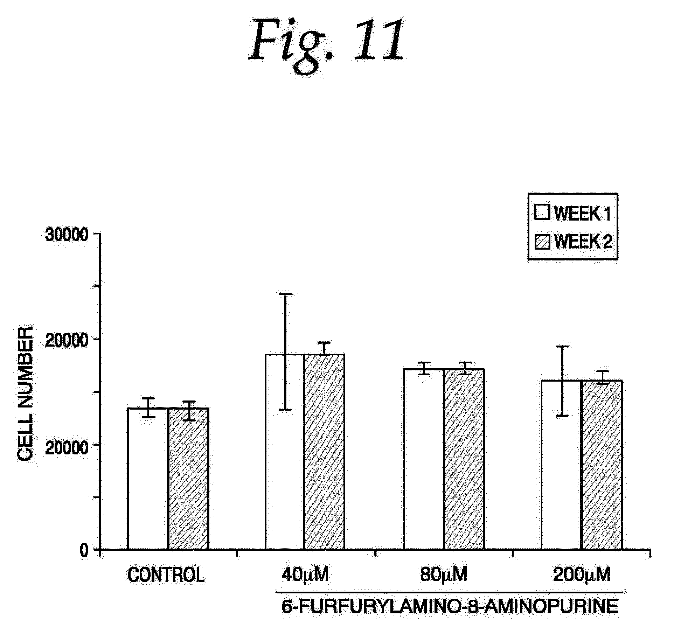

Effects on young and senescent cells. (1) Cytoskeletal organization: Early passage cells with less than 30% lifespan were treated with different doses of 6-furfurylamino-8-aminopurine (**82**) in order to see if this treatment could revert any of the age related changes. Figure 9 shows actin staining pattern in young cells after 3 days. There are no obvious differences in treated and untreated cells. (2) Morphology of senescent cells: Senescent human skin fibroblasts were treated with various doses of 6-furfurylamino-8-aminopurine to see if this treatment could reverse age-related alterations in their morphology. Figure 10 shows that there were significant differences in the appearance of cells after 7 days and 14 days of treatment. After about 90% lifespan, the cells progressively become heterogeneous with large and flattened appearance. We observed that cells grown in the presence of 6-furfurylamino-8-aminopurine were looking relatively younger in terms of their parallel arrangement after 14 days of treatment. At doses (40 and 80 µM) the cells appeared better with less intracellular debris than the controls. Therefore, it appears that 6-furfurylamino-8-aminopurine is well tolerated by young and old senescent cells, and can have some reversion effect on senescent human fibroblasts. Figure11 shows that 6-furfurylamino-8-aminopurine maintains the number of surviving senescent cells within 7 to 14 days. This rules out the negative effects of 6-furfurylamino-8-aminopurine and suggests that senescent cells were able to survive when treated with 6-furfurylamino-8-aminopurine.

The results obtained so far from these series of experiments on the effects of 6-furfurylamino-8-aminopurine on human skin fibroblasts suggests that this compound has beneficial anti-ageing and cosmetic effects on dermal fibroblasts and should have similar effects on human skin.

**Example 15: Ames Test.** A number of tests related to the safety of these inventive compounds have been carried out using conventionally accepted protocols and procedures. Tests were carried out using DMSO as solvent and dose levels of 8-amino-6-furfurylaminopurine at 2.5, 5.0, 15, 50, 500, 1500, and 5000 µg/plate based upon standard protocol and procedures (Ames et al., Mutation Research, 31, 347-364 (1975); Maron et al., Mutation Research, 113, 173-215 (1983)). Using *Salmonella typhimurium* histidien auxotrophs TA98 and TA100 in the presence and absence of Aroclor-induced rat liver S9, no positive mutagenic responses were observed with 8-amino-6-furfurylaminopurine.

**Example 16: Evaluation of cutaneous effects of new topical compounds in the hirless mouse assay.** The purpose of this study was to evaluate new topical compounds for their safety and efficacy as topical skin anti-aging treatments. The hairless mouse model is a well established model for studying the treatments of photoaging. This model has been used to study mechanisms, define effective treatment regimens and assess safety for retinoids and other anti-aging products. The present study was used to investigate a new class of anti-aging compounds in comparison to classical ones. Recent clinical studies have shown that this class of compounds improve the appearance of photodamaged skin and decrease transepidermal water loss (TEWL) without causing skin irritation. This study investigated the mechanisms of how these compounds affect the skin aging process and help to identify new compounds which are safe and effective for preventing and improving the clinical features of photoaged skin. Topical compounds and vehicle controls were be applied daily (Monday-Friday) for 3 weeks to the dorsal skin of hairless mice. At baseline (prior to the 1st treatment), and weekly, the dorsal skin was measured for: transepidermal water loss (TEWL); skin moisture content; and skin elasticity.

This study determined the effect of topical compound formulations on epidermal cell proliferation using bromodeoxyuridine as an immunohistochemical marker of cell proliferation. Histological examination of the treated and control skin were used to determine cutaneous effects of the topical compounds. Topical tretinoin 0.05% (Renova™) was used as a therapeutic control.

Experimental Design. 48 female SKH-1 hairless mice (5 weeks old, 20-25 grams, Charles River Laboratories, Wilmington, MA) are individually housed in filter-top cages, and acclimated for 5-7 days after delivery. The mice are divided into 8 treatment groups (n=6), including two different control groups (untreated control, vehicle control) and a therapeutic control (tretinoin 0.05% Renova™ cream). Cage cards are marked to indicate treatment groups 1-8. Water and mouse chow are provided *ad libitum.*

The experimental treatment of test groups are shown below; each group contained 6 test samples:

| **Group Number** | **Type** | **Treatment (20µl dose)** |
|---|---|---|
| 1 | Untreated Control | No Treatment |
| 2 | Vehicle Control | Vehicle Only |
| 3 | Kinetin Control | Vehicle + Kinetin (0.1%) |
| 4 | Zeatin Control | Vehicle + Zeatin (0.1%) |
| 5 | Kinetin/Zeatin Control | Vehicle + Kinetin (0.1%) + Zeatin (0.1%) |
| 6 | Inventive Compound (**82**) | Vehicle + 6-Furfurylamino-8-amino purine (0.1%) |
| 7 | 6-Furfurylamino-9-(2-tetrahydropyranyl) purine Control | Vehicle + 6-Furfurylamino-9-(2-tetrahydropyranyl)purine (0.1%) |
| 8 | Therapeutic Control | Renova™ (0.05% tretinoin cream) |

Erythema Scoring. Daily examinations are performed to assess the possible occurrence of erythema (irritation) of the sites using established scoring criteria:

| Erythema | Score Appearance |
|---|---|
| 0 | No response |
| 1 | Very slight redness |
| 2 | Slight redness |
| 3 | Moderate redness/irritation |
| 4 | Severe redness/irritation |
| 5 | Very severe redness/irritation |
| 6 | Necrosis |

Technical staff recorded erythema scores for all animals each morning. Should any animal meet or exceed a score of 4 for erythema, treatments for that animal was discontinued, and the animal was sacrificed for biopsy. Photographs were taken of selected animals to document the degree of skin irritation. At baseline (i.e., prior to the 1st application of test product) and weekly thereafter, the objective measurements were made on the dorsal skin site.

Transepidermal Water Loss (TEWL). The measurement of transepidermal water loss (TEWL) is an important noninvasive method used to characterize the effects of moisturizers on skin barrier function. A ServoMed Evaporimeter (Model EP-2) was used to measure the evaporative water loss at the skin treatment site or untreated control skin at baseline (prior to the 1st application), and at weekly intervals. The measurements are taken by placing a probe on the skin surface. Measurements of TEWL are then recorded to an integrated computer. Mean TEWL measurements was determined for each treatment group over time, and comparative statistics used to evaluate product effectiveness.

Skin Moisture Content & Elasticity. The measurements of skin moisture content and skin elasticity are important noninvasive methods used to characterize the effects of moisturizers and anti-wrinkle effects on skin. A DermaLab™ combination instrument were used to measure the skin moisture content and elasticity of the target skin sites at baseline and at weekly intervals. This instrument is equipped with dual probes which are placed on the skin surface and a quantitative measurement taken of the respective parameters and the measurements recorded on an integrated computer. Mean measurements was determined for each treatment group over time, and comparative statistics used to evaluate product effectiveness.

Topical Treatments. Beginning on Treatment Day 1, test products are applied topically each morning (Monday through Friday) after erythema evaluations for 3 weeks. Using a positive displacement pipette, 20mg of each agent is applied to a 2 cm x 2 cm area of the center midline of the back. The material was spread across the treatment area with the pipette tip.

Bromodeoxyuridine Injection. All animal groups are injected with bromodeoxyuridine (100mg/kg) I.P. 4 hours after the final application. Animals are sacrificed by CO2 inhalation 3 hours later followed by cervical dislocation. The test sites are excised, and 6 mm punch biopsies are obtained from each treatment site, and from untreated controls. Biopsies are placed into labeled vials containing 4% neutral buffered formalin for paraffin embedding and anti-BrdU staining.

Bromodeoxyuridine Imunohistochemical Staining. Paraffin sections are cut to a 5 uM thickness and stained using the BrdU Immuno-histochemistry kit (X1545K from Exalpha Biologicals, Inc.) and a standard staining protocol. The slides are weakly counterstained in Mayer's hematoxylin and scored under a light microscope for the number of BrdU-positive cells per mm of epidermis for each section. The data are expressed as mean BrdU-positive cells/mm for each treatment group. Differences in BrdU labeling in the treatment groups are statistically analyzed using the Students t-test. Representative photographs are taken of the histological sections of the BrdU-positive staining cells. Skin Histology. Skin biopsies are taken of each treatment site and untreated control skin. Biopsies are fixed in 4% neutral buffered formalin, embedded in paraffin, and stained with hematoxylin and eosin. The stained skin sections are examined to determine the effects of the treatment on epidermal, dermal, and stratum corneum histology. Biopsies are also microscopically examined for inflammatory cells.

Skin Compartment Measurements. The H&E stained biopsy slides were imaged using a trinocular Olympus BH-2 microscope fitted with a Microfire 2.1 x 3 Digital Camera. 100x images were digitized and stored on CD using MS Pictureframe and Microfire Software. All compartment images were measured using a calibrated micrometer. An average of three measurements of each compartment for two slide images per biopsy was obtained and tabulated, and means and standard deviations were calculated.

### Results.

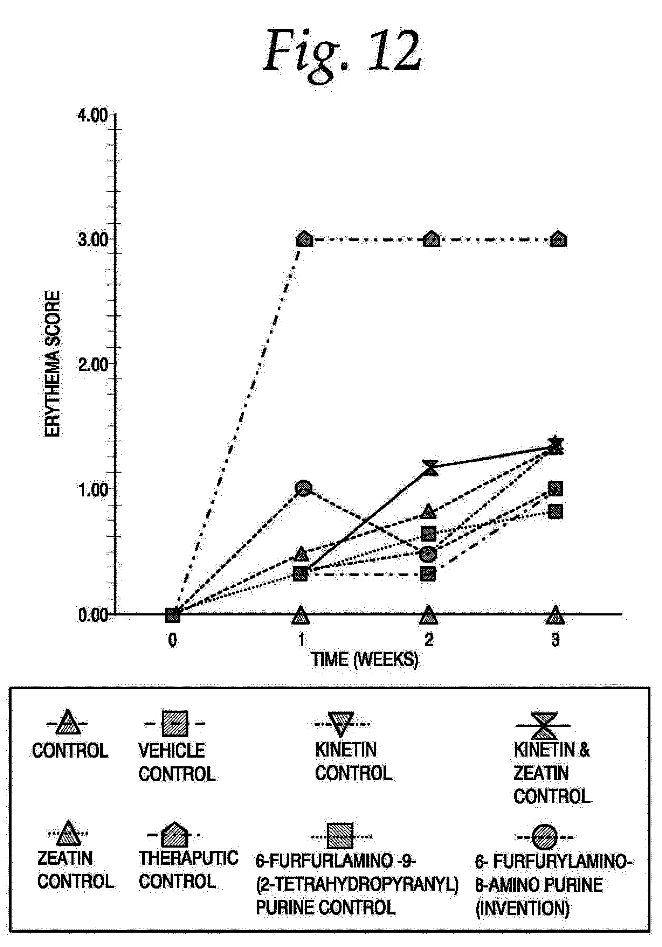

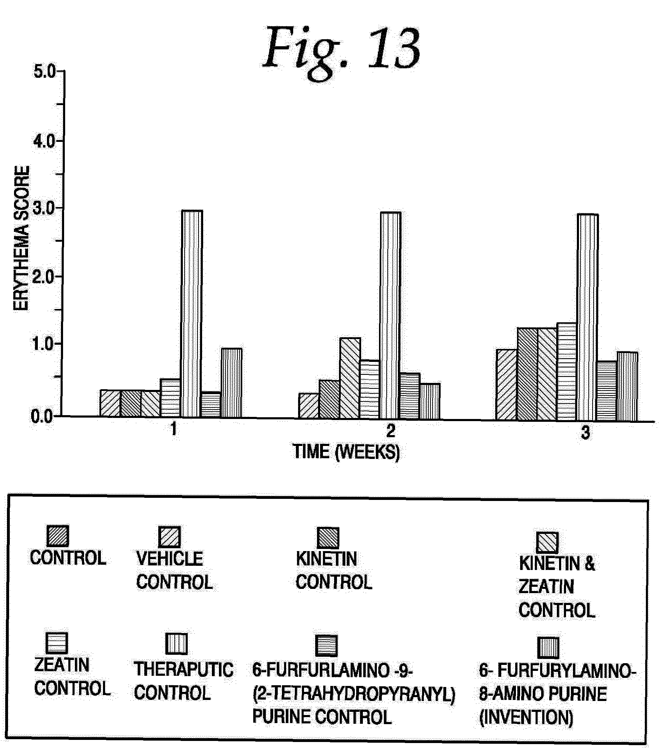

Skin Irritation. The topical compounds were well tolerated with extended treatment for 3 weeks. The tretinoin cream (Renova®) caused significant irritation (Grade 3) following 1 to 3 weeks of treatment. The results (Figs. 12 and 13) show that there is a gradual increase in erythema with the vehicle and the test compounds, but this was very slight (Grade 1). Some irritation may be expected with a cream vehicle.

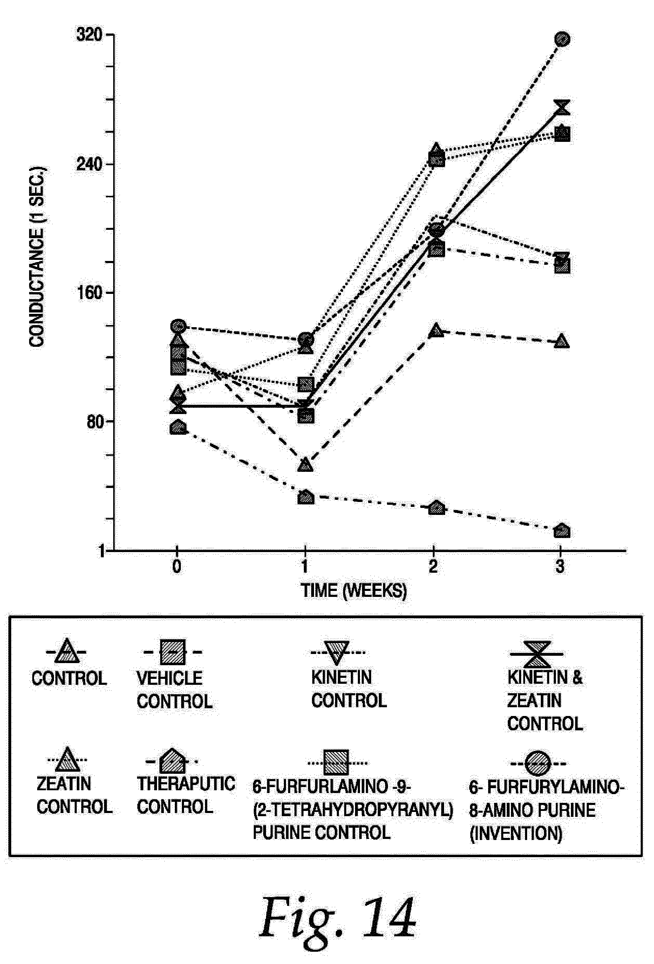

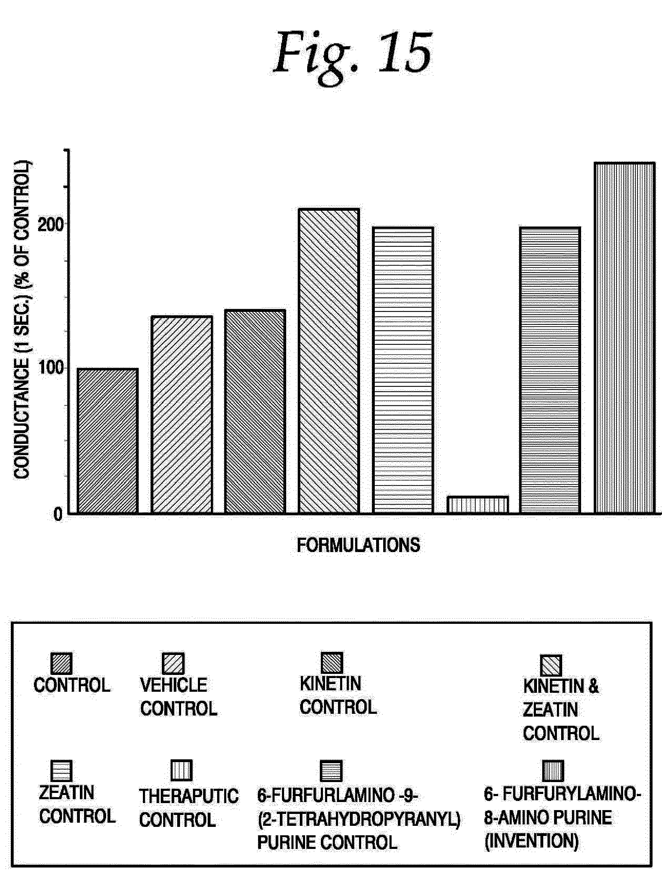

Skin Moisture Content. The therapeutic control (tretinoin) showed a significant decrease in skin conductance (Figure 14) which is a measure of the moisture content of the skin. In contrast, the topical compounds produced a gradual increase in skin moisture content. The vehicle also produced a gradual increase in moisture content, however, the average moisture content with 6-Furfurylamino-9-(2-tetrahydropyranyl) purin, Inventive Compound **82,** Kinetin + Zeatin, and Zeatin alone, was higher than that of the vehicle. These findings are consistent with the clinical experience with Kinetin which shows a decrease in TEWL, reflected by moisture retention, and an increase in TEWL with tretinoin. At week 3 the mean moisture content of the all topical compounds was greater than the vehicle or untreated control (Figure 15). The inventive compound 6-furfurylamino-8-aminopurine (compound **82**) was the most effective.

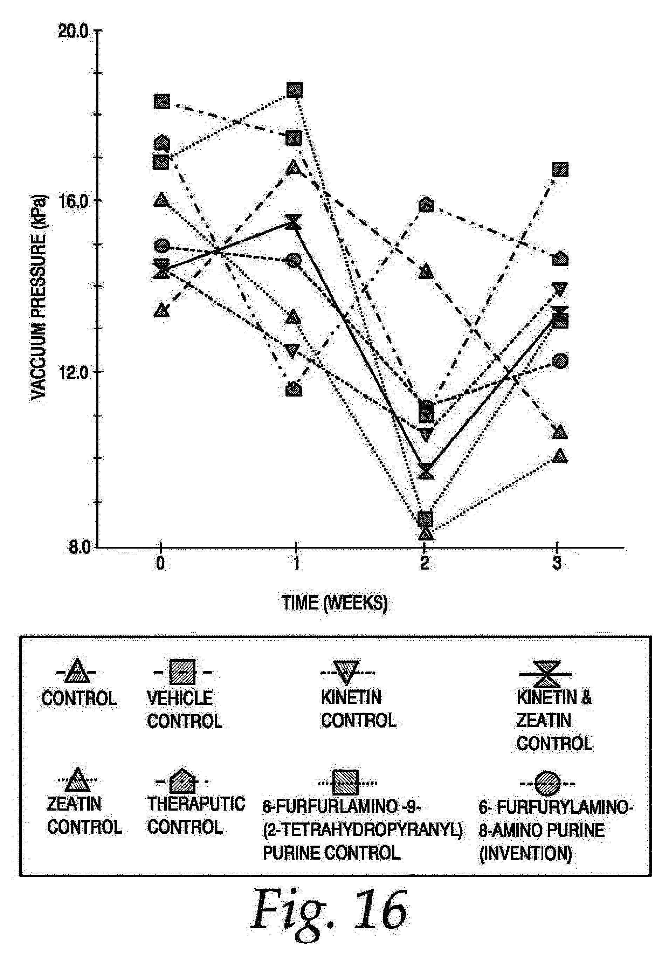

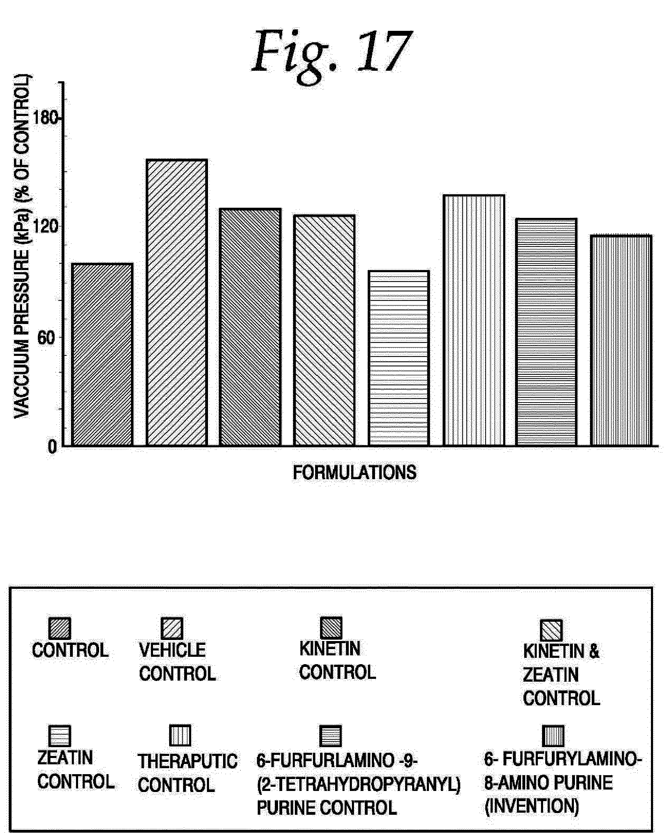

Skin Elasticity. There were no significant changes in the skin elasticity of the treatment groups. Such changes in the dermal structures may require an increased treatment period. The skin elasticity of the treatment groups was comparable to that of the untreated control and vehicle treatment (Figs. 16 and 17). Inventive Compound **82** was better than controls tested.

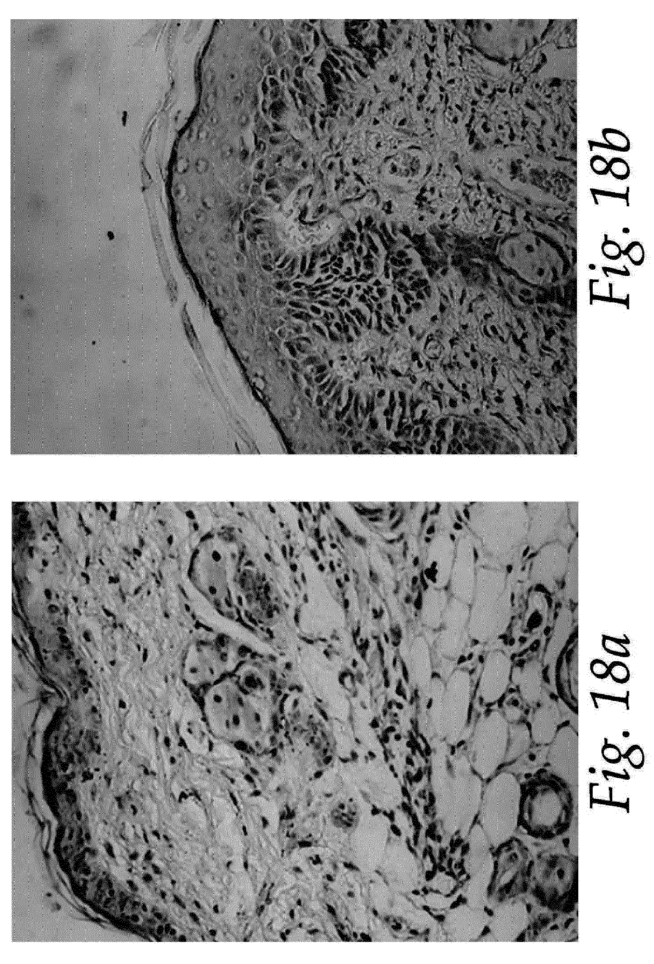

Transepidermal Water Loss (TEWL). Technical problems with the ServoMed evaporimeter precluded obtaining accurate measurements of TEWL. Previous clinical studies have demonstrated a decrease in TEWL with kinetin treatment. The increased moisture content of the skin in the treated mice would be consistent with these findings. Bromodeoxyuridne (BrdU) Staining. Bromodeoxyuridine staining of the epidermis was measured to determine the effect of the compounds on epidermal cell proliferation. There was no statistical difference in epidermal BrdU staining in the topical compounds compared to the untreated or vehicle control or any differences in BrdU staining with the different compounds. The tretinoin-treated tissues had no epidermal BrdU staining, but some localized areas of staining in the dermis, possibly related to the retinoid-induced inflammation. Inventive compound **82.** was again more effective (see Table 7 below and Figure 18).

**Table 7. Mean epidermal BrdU staining**

| **Group Number** | **Type** | **Mean Nuclei Stained (mm)** |
|---|---|---|
| 1 | Untreated Control | 5.8 ± 3.2 |
| 2 | Vehicle Control | 5.3 ± 3.9 |
| 3 | Kinetin Control | 2.4 ± 2.0 |
| 4 | Zeatin Control | 3.5 ± 3.4 |
| 5 | Kinetin/Zeatin Control | 1.9 ± 2.2 |
| 6 | Inventive Compound (82) | 12.8 ± 13.3 |
| 7 | 6-Furfurylamino-9-(2-tetrahydropyranyl) purine Control | 5.3 ± 4.2 |
| 8 | Therapeutic Control | 0 |

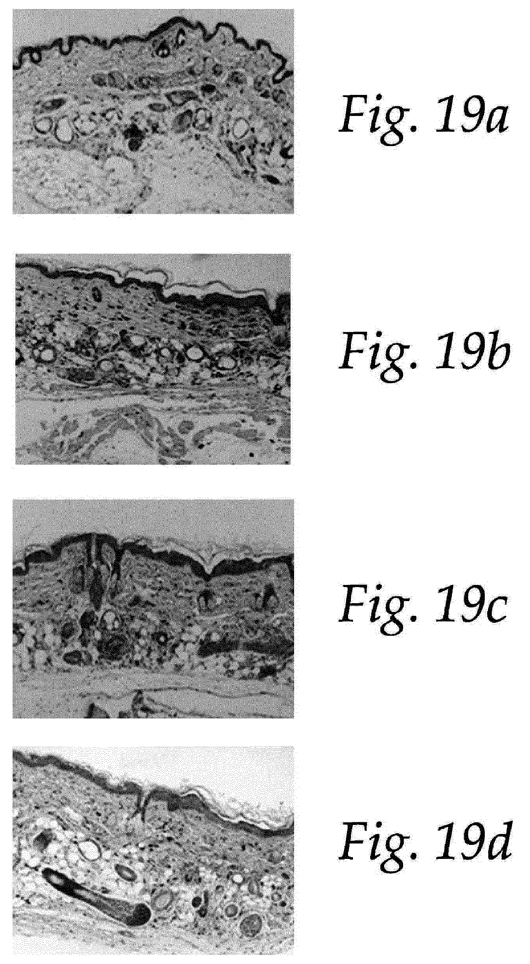

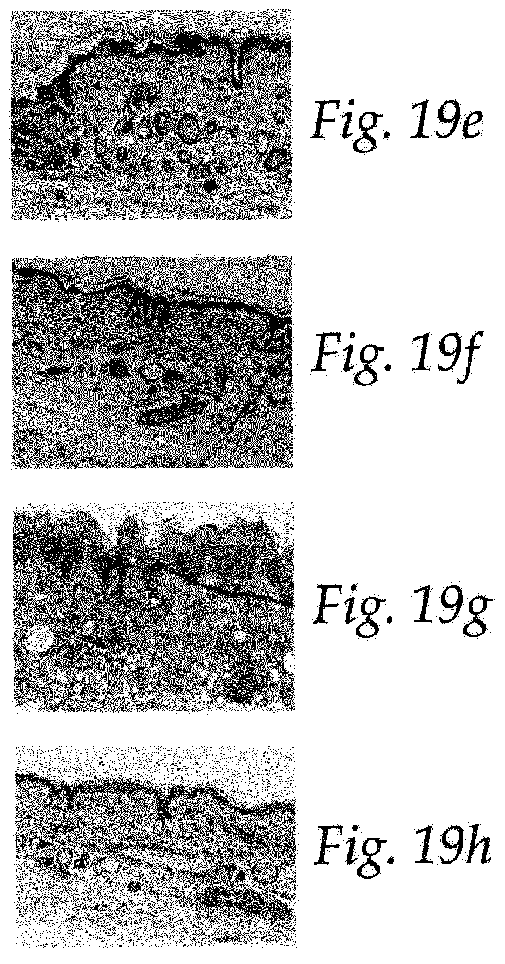

Skin Histology. Tissue biopsies were obtained at the completion of the study after 3 weeks of treatment (Figure 19). The histological evaluation showed normal "healthy" appearing skin, with all topical compounds. In contrast, the therapeutic control (tretinoin) showed marked increased thickness of the epidermis and inflammatory changes in the dermis. The skin compartment thickness of the H&E stained biopsies was measured by optical microscopy. The thickness of the epidermis, dermis and stratum corneum measured after 3 weeks of treatment was comparable to that of the vehicle and untreated control. In contrast, the therapeutic control (tretinoin) increased both epidermal and dermal thickness. Inventive compound **82** had the least effect on skin thickness from the active ingredients tested.

In summary, the overall provide evidence for both the safety and efficacy of the 6,8-disubstitued purine inventive compounds for the safety and efficacy for skin aging. This established model for cutaneous safety and skin aging has demonstrated that the 6,8-disubstitued purine inventive compounds has the potential to improve the appearance of skin aging without irritation, commonly produced by other anti-aging products. This study complements the results of the cell culture and human skin irritation results and provide support for the future clinical development of the inventive compounds for skin anti-aging and cosmetic applications.

**EXAMPLE 17: Formulations.** The growth regulatory formulations usually contain from 0.1 to 99% by weight, especially from 0.1 to 95% by weight, of active ingredient mixture comprising a 6,8-disubstitued purine of this invention, from 1 to 99.9% by weight of a solid or liquid formulation adjuvant, and from 0 to 25% by weight, especially from 0.1 to 25% by weight, of a surfactant. Whereas commercial products are usually formulated as concentrates, the end user will normally employ dilute formulations. The compositions may also comprise further ingredients, such as stabilizers, e.g., vegetable oils or epoxidised vegetable oils (epoxidised coconut, rapeseed oil or soybean oil), antifoams, e.g., silicone oil, preservatives, viscosity regulators, binders, tackifiers, and also fertilisers or other active ingredients. Preferred formulations have especially the following compositions: (% = percent by weight):

| Emulsifiable concentrates | | a) | b) | | c) | d) |
|---|---|---|---|---|---|---|
| active ingredient mixture | | 5% | 10% | | 25% | 50% |
| calcium dodecylbenzenesulfonate | | 6% | 8% | | 6% | 8% |
| castor oil polyglycol ether (36 mol of ethylene oxide) | 4% | - | | 4% | 4% | |
| octylphenol polyglycol ether (7-8 mol of ethylene oxide) | - | 4% | | - | 2% | |
| cyclohexanone | | - | - | | 10% | 20% |
| aromatic hydrocarbon mixture (C9-C12) | 85% | 78% | | 55% | | 16% |

Emulsions of any desired concentration can be obtained from such concentrates by dilution with water.

| Solutions | a) | b) | c) | d) |
|---|---|---|---|---|
| active ingredient mixture | 5% | 10% | 50% | 90% |
| 1-methoxy-3-(3-methoxy-propoxy)-propane | - | 20% | 20% | - |
| polyethylene glycol (MW 400) | 20% | 10% | - | - |
| N-methyl-2-pyrrolidone | - | - | 30% | 10% |
| aromatic hydrocarbon mixture 9C₉-C₁₂) | 75% | 60% | - | - |

The solutions are suitable for use in the form of microdrops.

| Wettable powders | a) | b) | c) | d) |
|---|---|---|---|---|
| active ingredient mixture | 5% | 25% | 50% | 80% |
| sodium lignosulfonate | 4% | - | 3% | - |
| sodium lauryl sulfate | 2% | 3% | - | 4% |
| sodium diisobutylnaphthalene-sulfonate | - | 6% | 5% | 6% |
| octylphenol polyglycol ether (7-8 mol of ethylene oxide) | - | 1% | 2% | - |
| highly dispersed silicic acid | 1% | 3% | 5% | 10% |
| kaolin | 88% | 62% | 35% | - |

The active ingredient is mixed thoroughly with the adjuvants and the mixture is thoroughly ground in a suitable mill, affording wettable powders which can be diluted with water to give suspensions of any desired concentration.

| Coated granules | a) | b) | c) |
|---|---|---|---|
| active ingredient mixture | 0.1% | 5% | 15% |
| highly dispersed silicic acid | 0.9% | 2% | 2% |
| inorganic carrier (AE 0.1-1 mm; e.g., CaCO₃ or SiO₂) | 99.0% | 93% | 83% |

The active ingredient is dissolved in methylene chloride and applied to the carrier by spraying, and the solvent is then removed by evaporation using vacuum.

| Coated granules | a) | b) | c) |
|---|---|---|---|
| active ingredient mixture | 0.1% | 5% | 15% |
| polyethylene glycol MW 200 | 1.0% | 2% | 3% |
| highly dispersed silicic acid | 0.9% | 1% | 2% |
| inorganic carrier (AE 0.1 -1 mm; e.g., CaCO₃ or SiO₂) | 98.0% | 92% | 80% |

The finely ground active ingredient is uniformly applied, in a mixer, to the carrier moistened with polyethylene glycol. Non-dusty coated granules are obtained in this manner.

| Extruder granules | a) | b) | c) | d) |
|---|---|---|---|---|
| active ingredient mixture | 0.1% | 3% | 5% | 15% |
| sodium lignosulfonate | 1.5% | 2% | 3% | 4% |
| carboxymethylcellulose | 1.4% | 2% | 2% | 2% |
| kaolin | 97.0% | 93% | 90% | 79% |

The active ingredient is mixed and ground with the adjuvants, and the mixture is moistened with water. The mixture is extruded and then dried in a stream of air.

| Dusts | a) | b) | c) |
|---|---|---|---|
| active ingredient mixture | 0.1% | 1% | 5% |
| talcum | 39.9% | 49% | 35% |
| kaolin | 60.0% | 50% | 60% |

Ready-to-use dusts are obtained by mixing the active ingredient with the carriers and grinding the mixture in a suitable mill.

| Suspension concentrates | a) | b) | c) | d) |
|---|---|---|---|---|
| active ingredient mixture | 3% | 10% | 25% | 50% |
| ethylene glycol | 5% | 5% | 5% | 5% |
| nonylphenol polyglycol ether (15 mol of ethylene oxide) | - | 1% | 2% | - |
| sodium lignosulfonate | 3% | 3% | 4% | 5% |
| carboxymethylcel lulose | 1% | 1% | 1% | 1% |
| 37% aqueous formaldehyde | 0.2% | 0.2% | 0.2% | 0.2% |
| silicone oil emulsion | 0.8% | 0.8% | 0.8% | 0.8% |
| water | 87% | 79% | 62% | 38% |

The finely ground active ingredient is intimately mixed with the adjutants, giving a suspension concentrate from which suspensions of any desired concentration can be obtained by dilution with water.

Dry Capsules. 5000 capsules, each of which contain 0.25 g of one of the 6,8-disubstitued purineas active ingredient, are prepared as follows:
Composition: Active ingredient: 1250 g; Talc:180 g; Wheat starch:120 g; Magnesium stearate:80 g; Lactose 20 g.
Preparation process: The powdered substances mentioned are pressed through a sieve of mesh width 0.6 mm. Portions of 0.33 g of the mixture are transferred to gelatine capsules with the aid of a capsule-filling machine.

Soft Capsules. 5000 soft gelatine capsules, each of which contain 0.05 g of one of the 6,8-disubstitued purine as active ingredient, are prepared as follows:
Composition: 250 g Active ingredient + 2 litres Lauroglycol.
Preparation process: The powdered active ingredient is suspended in Lauroglykol^{®} (propylene glycol laurate, Gattefossé S.A., Saint Priest, France) and ground in a wet-pulveriser to a particle size of about 1 to 3 mm. Portions of in each case 0.419 g of the mixture are then transferred to soft gelatine capsules by means of a capsule-filling machine.

Soft Capsules. 5000 soft gelatine capsules, each of which contain 0.05 g of one of the 6,8-disubstitued purine as active ingredient, are prepared as follows:
Composition: 250 g Active ingredient +1 litre PEG 400 +1 litre Tween 80.
Preparation process: The powdered active ingredient is suspended in PEG 400 (polyethylene glycol of Mr between 380 and about 420, Sigma, Fluka, Aldrich, USA) and Tween^{®} 80 (polyoxyethylene sorbitan monolaurate, Atlas Chem. Inc., Inc., USA, supplied by Sigma, Fluka, Aldrich, USA) and ground in a wet-pulveriser to a particle size of about 1 to 3 mm. Portions of in each case 0.43 g of the mixture are then transferred to soft gelatine capsules by means of a capsule-filling machine.

**EXAMPLE 18: Gel formulation.** An ointment formulation was tested during a pilot clinical study with 4 volunteers with psoriatic skin disorders. The components are given in grams per 100 g.

| **Compound** | **Content** |
|---|---|
| 8-Amino-6-furfurylaminopurine | 1.0 g |
| Butylhydroxytoluenum | 0.2 g |
| Butylparaben | 0.2 g |
| Diethyleneglycol monoethyl ether | 10.0 g |
| Silica colloidalis anhydrica | 5.0 g |
| Propylene glycol laurate | 83.6 g |

The gel consistence may be additionally modified by addition of silica colloidalis anhydrica. It is again expected that the transdermal Transcutol P/Lauroglycol FCC system will increase the efficiency of 8-amino-6-furfurylaminopurine. Silica colloidalis anhydrica will probably slow down the penetration of the active substance.

**EXAMPLE 19: Preparation procedure of a skin ointment.** The formulation components are given in grams per 200 g:

| **Compound** | **Content** |
|---|---|
| 8-Amino-6-furfurylaminopurine | 2.0 g |
| Butylhydroxytoluenum) | 0.4 g |
| Butylparaben | 0.4 g |
| Diethyleneglycol monoethyl ether) | 20.0 g |
| Glycerol dibehenate | 44.0 g |
| Propylene glycol laurate | 133.2 g |

Recommended procedure. Phase A: 2 grams of 8-amino-6-furfurylaminopurine were dissolved in 20 g of Transcutol P while stirring continuously at room temperature in a separate glass or stainless-steel container. The dissolution process may be accelerated by heating the solution to a maximal temperature of 40°C. Phase B: 0.4 grams of Nipanox BHT and 0.4 g of Nipabutyl were dissolved while stirring continuously in 133.2 g of Lauroglycol FCC at a temperature of approximately 70°C in another separate glass or stainless-steel container. The clear oily solution is heated to a temperature of approximately 80°C and 44 g of Compritol 888 ATO are melted in it while stirring continuously. The clear oily solution is cooled down to approximately 60°C and during continuous stirring and cooling down is mixed with phase A. The resulting whitish ointment-like substance is divided into approximately 15 gram portions and filled into prearranged plastic containers.

**Example 20. Formulation of a composition for topical application to the skin.** A composition for topical application to the skin contains the following ingredients by weight percent:

| | |
|---|---|
| Active ingredient: | |
| 8-Amino-6-furfurylaminopurine | 0.1 % |
| Oil phase: | |
| Cetyl alcohol | 5.0 % |
| Glyceryl monostearate | 15.0% |
| Sorbitan monooleate | 0.3 % |
| Polysorbate 80 USP | 0.3 % |
| Aqueous phase: | |
| Methylcellulose 100 cps | 1.0 % |
| Methyl paraben | 0.25 % |
| Propyl paraben | 0.15 % |
| Purified water | q.s. to 100 % |

Methyl paraben and propyl paraben were dissolved in hot water and subsequently methylcellulose was dispersed in the hot water. The mixture was chilled at 6C until the methylcellulose dissolved. The mixture was then heated to 72°C and added to the oil phase which was heated to 70°C while stirring continuously. 8-Amino-6-furfurylaminopurine was added at a temperature of 35°C and the resulting mixture was stirred continuously until dispersed. This composition is applied to the skin on at least a daily basis until the desired skin-ameliorating (anti-aging) or cosmetic effect is reached.

## Claims

1. 6,8-Disubstituted purines of general formula 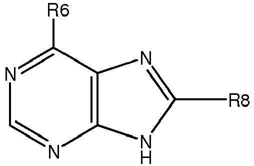 and salts thereof; wherein
R6 is -NH-R_{y}, R_{y} is selected from the group consisting of furfuryl, benzyl, 3-methylbut-2-en-1-yl, wherein R_{y} can be unsubstitued or substituted with one or more hydroxy, methoxy, and
R8 is selected from the group consisting of amino, amino(C₁-C₅ alkyl)amino, methoxy, alkyloxycarbonyl, and alkylamino.

2. The 6,8-disubstituted purines of claim 1, wherein R8 is amino.

3. The 6,8-disubstituted purines of claim 1, wherein the 6,8-disubstituted purines are 6-furfurylamino-8-(amino, amino(C₁-C₅ alkyl)amino, methoxy)purine, 6-(3-hydroxybenzylamino)-8-(amino, amino(C₁-C₅ alkyl)amino, methoxy)purine, 6-(4-hydroxybenzylamino)-8-(amino, amino(C₁-C₅ alkyl)amino, methoxy)purine, 6-(Z)-(4-hydroxy-3-methylbut-2-en-1-ylamino)-8-(amino, amino(C₁-C₅ alkyl)amino, methoxy)purine, 6-(E)-(4-hydroxy-3-methylbut-2-en-1-ylamino)-8-(amino, amino(C₁-C₅ alkyl)amino, methoxy)purine, or salts thereof.

4. The 6,8-disubstituted purines of claim 3, the 6,8-disubstituted purines are 8-amino-6-furfurylaminopurine or salts thereof wherein the furfuryl group can optionally be substituted with one or more hydroxy, methoxy, or combinations thereof.

5. The 6,8-disubstituted purines of claim 3, the 6,8-disubstituted purines are 8-amino-6-benzylaminopurine or salts wherein benzyl group can optionally be substituted with one or more hydroxy, methoxy, or combinations thereof.

6. Use of_6,8-disubstituted purines of general formula 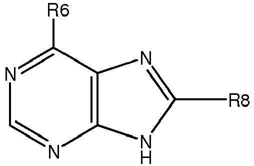 and salts thereof; wherein
R6 is -NH-R_{y}, R_{y} is selected from the group consisting of furfuryl, benzyl, 3-methylbut-2-en-1-yl, wherein R_{y} can be unsubstitued or substituted with one or more hydroxy, methoxy, and
R8 is selected from the group consisting of amino, bromo, amino(C₁-C₅ alkyl)amino, methoxy, alkyloxycarbonyl, and alkylamino,
for ameliorating adverse effect of aging in mammalian cells.

7. The use of claim 6, wherein the mammalian cells are human skin cells on a living human and wherein the 6,8-disubstituted purines are topically administered to the human skin cells.

8. The use of claim 7, wherein the 6,8-disubstituted purine is 8-amino-6-furfurylamino purine or salts thereof.

9. Use of 6,8-disubstituted purines of general formula 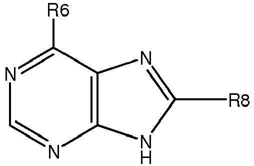 and salts thereof; wherein
R6 is -NH-R_{y}, R_{y} is selected from the group consisting of furfuryl, benzyl, 3-methylbut-2-en-1-yl, wherein R_{y} can be unsubstitued or substituted with one or more hydroxy, methoxy, and
R8 is selected from the group consisting of amino, bromo, amino(C₁-C₅ alkyl)amino, methoxy, alkyloxycarbonyl, and alkylamino,
for improving the cosmetic appearance of human skin.

10. The use of claim 9, wherein the 6,8-disubstituted purines are 6-furfurylamino-8-(amino, bromo, amino(C₁-C₅ alkyl)amino, methoxy)purine, 6-(3-hydroxybenzylamino)-8-(amino, bromo, amino(C₁-C₅ alkyl)amino, methoxy)purine, 6-(4-hydroxybenzylamino)-8-(amino, bromo, amino(C₁-C₅ alkyl)amino, methoxy)purine, 6-(Z)-(4-hydroxy-3-methylbut-2-en-1-ylamino)-8-(amino, bromo, amino(C₁-C₅ alkyl)amino, methoxy)purine, 6-(E)-(4-hydroxy-3-methylbut-2-en-1-ylamino)-8-(amino, bromo, amino(C₁-C₅ alkyl)amino, methoxy)purine, or salts thereof.

11. The use of claim 9, wherein the 6,8-disubstituted purine is 8-amino-6-furfurylamino purine or salt thereof.

## Patentansprüche

1. 6,8-disubstituierte Purine der allgemeinen Formel 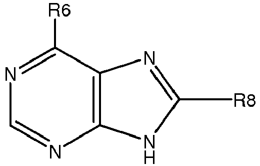 und deren Salze, worin
R6 bedeutet -NH-R_{y}, R_{y} aus der Gruppe ausgewählt ist, umfassend Furfuryl, Benzyl, 3-Methylbut-2-en-l-yl, wobei R_{y} unsubstituiert oder durch ein oder mehrere Hydroxy, Methoxy substituiert sein kann, und
R8 aus der Gruppe ausgewählt ist, bestehend aus Amino, Amino(C₁-C₅-Alkyl)amino, Methoxy, Alkyloxykarbonyl und Alkylamino.

2. 6,8-disubstituierte Purine nach dem Anspruch 1, worin R8 Amino bedeutet.

3. 6,8-disubstituierte Purine nach dem Anspruch 1, worin die 6,8-disubstituierten Purine sind 6-Furfurylamino-8-(amino, amino(C₁-C₅-Alkyl)amino, methoxy)purin, 6-(3-Hydroxybenzylamino)-8-(amino, amino(C₁-C₅-Alkyl)amino, methoxy)purin, 6-(4-Hydroxybenzylamino)-8-(amino, amino(C₁-C₅-Alkyl)amino, methoxy)purin, 6-(Z)-(4-Hydroxy-3-methylbut-2-en-1-ylamino)-8-(amino, amino(C₁-C₅-Alkyl)amino, methoxy)purin, 6-(E)-(4-Hydroxy-3-methylbut-2-en-1-ylamino)-8-(amino, amino(C₁-C₅-Alkyl)amino, methoxy)purin oder deren Salze.

4. 6,8-disubstituierte Purine nach dem Anspruch 3, worin die 6,8-disubstituierten Purine sind 8-Amino-6-furfurylaminopurin oder dessen Salze, worin die Furfurylgruppe allfällig durch ein oder mehrere Hydroxy, Methoxy oder deren Kombinationen substituiert sein kann.

5. 6,8-disubstituierte Purine nach dem Anspruch 3, worin die 6,8-disubstituierten Purine sind 8-Amino-6-benzylaminopurin oder dessen Salze, worin die Benzylgruppe allfällig durch ein oder mehrere Hydroxy, Methoxy oder deren Kombinationen substituiert sein kann.

6. Verwendung der 6,8-disubstituierten Purine der allgemeinen Formel 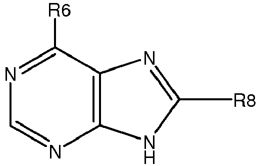 und deren Salze, worin
R6 bedeutet -NH-R_{y}, R_{y} ausgewählt ist aus der Gruppe, bestehend aus Furfuryl, Benzyl, 3-Methylbut-2-en-l-yl, wobei R_{y} unsubstituiert oder durch ein oder mehrere Hydroxy, Methoxy substituiert sein kann, und
R8 ausgewählt ist aus der Gruppe, umfassend Amino, Bromo, Amino(C₁-C₅-Alkyl)amino, Methoxy, Alkyloxykarbonyl und Alkylamino,
zur Verbesserung von ungünstigen Wirkungen der Alterungsprozesse in den Säugerzellen.

7. Verwendung nach dem Anspruch 6, wobei die Säugerzellen Zellen der menschlichen Haut beim lebenden Menschen sind und wobei die 6,8-disubstituierten Purine diesen Zellen der menschlichen Haut topisch verabreicht werden.

8. Verwendung nach dem Anspruch 7, wobei das 6,8-disubstituierte Purine das 8-Amino-6-furfurylaminopurin oder dessen Salze ist.

9. Verwendung der 6,8-disubstituierten Purine der allgemein Formel 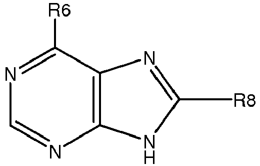 und deren Salze, worin
R6 bedeutet -NH-R_{y}, R_{y} ausgewählt ist aus der Gruppe, bestehend aus Furfuryl, Benzyl, 3-Methylbut-2-en-l-yl, wobei R_{y} unsubstituiert oder durch ein oder mehrere Hydroxy, Methoxy substituiert sein kann, und
R8 ausgewählt ist aus der Gruppe, bestehend aus Amino, Bromo, Amino(C₁-C₅-Alkyl)amino, Methoxy, Alkyloxykarbonyl und Alkylamino,
zur Verbesserung des kosmetischen Aussehens der menschlichen Haut.

10. Verwendung nach dem Anspruch 9, worbei die 6,8-disubstituierten Purine sind 6-Furfurylamino-8-(amino, bromo, amino(C₁-C₅-Alkyl)amino, methoxy)purin, 6-(3-Hydroxybenzylamino)-8-(amino, bromo, amino(C₁-C₅-Alkyl)amino, methoxy)purin, 6-(4-Hydroxybenzylamino)-8-(amino, bromo, amino(C₁-C₅-Alkyl)amino, methoxy)purin, 6-(Z)-(4-Hydroxy-3-methylbut-2-en-1-ylamino)-8-(amino, bromo, amino(C₁-C₅-Alkyl)amino, methoxy)purin, 6-(E)-(4-Hydroxy-3-methylbut-2-en-1-ylamino)-8-(amino, bromo, amino(C₁-C₅-Alkyl)amino, methoxy)purin oder deren Salze.

11. Verwendung nach dem Anspruch 9, wobei das 6,8-disubstituierte Purin das 8-Amino-6-furfurylaminopurin oder dessen Salz ist.

## Revendications

1. Purines 6,8-disubstituées de formule générale 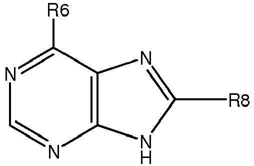 et leurs sels, dans laquelle
R6 est -NH-R_{y}, R_{y} est sélectionné parmi un groupe constitué de furfuryle, benzyle, 3-méthylbut-2-ène-1-yl, dans laquelle R_{y} peut être non substitué ou substitué par un ou plusieurs hydroxy, méthoxy, et
R8 est sélectionné parmi un groupe constitué de amino, amino (C₁-C₅ alkyl)amino, méthoxy, alkyloxycarbonyle, et alkylamino.

2. Purines 6,8-disubstituées selon la revendication 1, **caractérisées en ce que** R8 est amino.

3. Purines 6,8-disubstituées selon la revendication 1, **caractérisées en ce que** les purine 6,8-disubstituées sont 6-furfurylamino-8-(amino, amino(C₁-C₅ alkyl)amino, méthoxy)purine, 6-(3-hydroxybenzylamino)-8-(amino, amino(C₁-C₅ alkyl)amino, méthoxy)purine, 6-(4-hydroxybenzylamino)-8-(amino, amino(C₁-C₅ alkyl)amino, méthoxy)purine, 6-(Z)-(4-hydroxy-3-méthylbut-2-ène-1-ylamino)-8-(amino, amino(C₁-C₅ alkyl)amino, méthoxy)purine, 6-(E)-(4-hydroxy-3-méthylbut-2-ène-1-ylamino)-8-(amino, amino(C₁-C₅ alkyl)amino, méthoxy)purine, ou leurs sels.

4. Purines 6,8-disubstituées selon la revendication 3, **caractérisées en ce que** les purine 6,8-disubstituées sont 8-amino-6-furfurylaminopurine ou ses sels, le groupe furfuryle pouvant éventuellement être substitué par un ou plusieurs hydroxy, méthoxy, ou leurs combinaisons.

5. Purines 6,8-disubstituées selon la revendication 3, **caractérisées en ce que** les purine 6,8-disubstituées sont 8-amino-6-benzylaminopurine ou ses sels, le groupe benzyle pouvant éventuellement être substitué par un ou plusieurs hydroxy, méthoxy, ou leurs combinaisons.

6. Utilisation des purine 6,8-disubstituées de formule générale 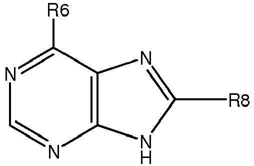 et de leurs sels, dans laquelle
R6 est -NH-R_{y}, R_{y} est sélectionné parmi un groupe constitué de furfuryle, benzyle, 3-méthylbut-2-ène-1-yl, dans laquelle R_{y} peut être non substitué ou substitué par un ou plusieurs hydroxy, méthoxy, et
R8 est sélectionné parmi un groupe constitué de amino, bromo, amino(C₁-C₅ alkyl)amino, méthoxy, alkyloxycarbonyle, et alkylamino,
pour l'amélioration des effets défavorables du vieillissement dans les cellules mammaliennes.

7. Utilisation selon la revendication 6, **caractérisée en ce que** les cellules mammaliennes sont des cellules de la peau humaine sur un humain vivant et **en ce que** les purines 6,8-disubstituées sont administrées à ces cellules de la peau humaine par voie topique.

8. Utilisation selon la revendication 7, **caractérisée en ce que** la purine 6,8-disubstituée est 8-amino-6-furfurylaminopurine ou ses sels.

9. Utilisation des purine 6,8-disubstituées de formule générale 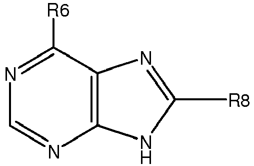 et de leurs sels, dans laquelle
R6 est -NH-R_{y}, R_{y} est sélectionné parmi un groupe constitué de furfuryle, benzyle, 3-méthylbut-2-ène-1-yl, dans laquelle R_{y} peut être non substitué ou substitué par un ou plusieurs hydroxy, méthoxy, et
R8 est choisi parmi un groupe constitué de amino, bromo, amino(C₁-C₅ alkyl)amino, méthoxy, alkyloxycarbonyle, et alkylamino,
pour l'amélioration de l'apparence cosmétique de la peau humaine.

10. Utilisation selon la revendication 9, **caractérisée en ce que** les purines 6,8-disubstituées sont 6-furfurylamino-8-(amino, bromo, amino(C₁-C₅ alkyl)amino, méthoxy)purine, 6-(3-hydroxybenzylamino)-8-(amino, bromo, amino(C₁-C₅ alkyl)amino, méthoxy)purine, 6-(4-hydroxybenzylamino)-8-(amino, bromo, amino(C₁-C₅ alkyl)amino, méthoxy)purine, 6-(Z)-(4-hydroxy-3-méthylbut-2-ène-1-ylamino)-8-(amino, bromo, amino(C₁-C₅ alkyl)amino, méthoxy)purine, 6-(E)-(4-hydroxy-3-méthylbut-2-ène-1-ylamino)-8-(amino, bromo, amino(C₁-C₅ alkyl)amino, méthoxy)purine, ou leurs sels.

11. Utilisation selon la revendication 9, **caractérisée en ce que** la purine 6,8-disubstituée est 8-amino-6-furfurylaminopurine ou son sel.
